(19) Europäisches Patentamt / European Patent Office / Office européen des brevets

(11) **EP 4 238 546 A1**

(12) **EUROPEAN PATENT APPLICATION**
published in accordance with Art. 153(4) EPC

(43) Date of publication:
**06.09.2023 Bulletin 2023/36**

(21) Application number: **21886310.8**

(22) Date of filing: **28.10.2021**

(51) International Patent Classification (IPC):
*A61K 6/887* (2020.01)        *A61K 6/15* (2020.01)
*A61K 6/16* (2020.01)        *A61K 6/17* (2020.01)
*A61K 6/30* (2020.01)        *A61K 6/60* (2020.01)
*A61K 6/65* (2020.01)        *A61K 6/76* (2020.01)
*A61K 6/84* (2020.01)

(52) Cooperative Patent Classification (CPC):
**A61K 6/15; A61K 6/16; A61K 6/17; A61K 6/30;
A61K 6/60; A61K 6/65; A61K 6/76; A61K 6/84;
A61K 6/887**

(86) International application number:
**PCT/JP2021/039803**

(87) International publication number:
**WO 2022/092193 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.10.2020 JP 2020180960**

(71) Applicant: **Kuraray Noritake Dental Inc.
Kurashiki-shi, Okayama 710-0801 (JP)**

(72) Inventors:
• **KAJIKAWA, Tatsuya
  Tainai-shi, Niigata 959-2653 (JP)**
• **HORIGUCHI, Hirotaka
  Tainai-shi, Niigata 959-2653 (JP)**
• **KAMEYA, Takehiro
  Tainai-shi, Niigata 959-2653 (JP)**

(74) Representative: **D Young & Co LLP
120 Holborn
London EC1N 2DY (GB)**

(54) **DENTAL CURABLE COMPOSITION HAVING GOOD COLOR COMPATIBILITY**

(57)    The present invention provides a dental curable composition that enables a highly aesthetic restoration that blends in so well with the surroundings that it is almost indistinguishable from natural teeth, and that, despite being a single dental curable composition, shows good color compatibility with natural teeth over a wide range of shades, particularly in cases involving cavity floors in different classes of cavities, such as Class I, Class II, and Class V. The present invention relates to a dental curable composition comprising a polymerizable monomer (A), a filler (B), a polymerization initiator (C), and a colorant (D), wherein the ratios $R_{650/600}$, $R_{700/600}$, and $R_{750/600}$ of spectral reflectances at 650 nm, 700 nm, and 750 nm wavelengths to a spectral reflectance at 600 nm wavelength all fall within a range of 97% to 103% when measured for a 1.0 mm-thick cured product of the dental curable composition against a white background with a spectral colorimeter.

EP 4 238 546 A1

**Description**

TECHNICAL FIELD

The present invention relates to dental materials that can replace a part or all of a natural tooth in the field of dentistry, particularly a dental curable composition that can be suitably used in applications such as dental composite resins.

BACKGROUND ART

Before the advent of dental curable compositions, particularly dental composite resins, materials such as amalgam and gold alloys were used for the filling treatment of dental caries. On the other hand, dental composite resins have rapidly gained popularity due to their affordability and the ability to relatively easily achieve shades close to natural teeth. Dental restorative filling materials have improved their mechanical strength and adhesion to teeth over the last years, and are now used not only for front teeth but also for other teeth, such as molars, where high occlusal pressure is applied. At present, dental restorative filling materials are used in the vast majority of filling treatments.

To date, various studies have been made to bring the appearance of dental composite resins closer to natural teeth. For example, Patent Literature 1 proposes a dental composite material that, in addition to using a base filler having a refractive index close to that of a cured product of a polymerizable monomer, contains another type of filler differing in refractive index from a cured product of the polymerizable monomer and having an average particle diameter of 1 $\mu$m or more, in order to moderately diffuse light that has entered a cured product of a composition containing the base filler and the polymerizable monomer. Such dental composite materials have a degree of diffusion for specific transmitted light, and can provide a highly aesthetic restoration because of light diffusion properties similar to the light diffusion properties of natural teeth. However, despite being similar in light diffusion properties to natural teeth, such dental composite materials involve a number of problems in clinical applications. For example, the shade of natural teeth differs from person to person, and even the teeth of the same individual have different shades in different parts of teeth. If the dental composite resin proposed in Patent Literature 1 were to be used to achieve a restoration with high aesthetics, it would be necessary to prepare a plurality of dental composite resins in different shades, and choose one that best matches the shade of actual teeth.

Choosing the best shade is a highly skillful technique, and it is not easy to match shades. From a dentists' perspective, there is a need to keep stock of dental composite resins of different shades, and this is a cost disadvantage.

CITATION LIST

Patent Literature

Patent Literature 1: JP H09-255516 A

SUMMARY OF INVENTION

Technical Problem

The present invention has been made to solve the problems involved in related art, and an object of the present invention is to provide a dental curable composition that enables a highly aesthetic restoration that blends in so well with the surroundings that it is almost indistinguishable from natural teeth, and that, despite being a single dental curable composition, shows good color compatibility with natural teeth over a wide range of shades. Another object of the present invention is to provide a dental curable composition that, despite being a single dental curable composition, shows good color compatibility with natural teeth over a wide range of shades, particularly in cases involving cavity floors in different classes of cavities, such as Class I, Class II, and Class V.

Solution to Problem

The present inventors conducted intensive studies to achieve the foregoing objects, and found that a dental curable composition shows good color compatibility with natural teeth over a wide range of shades when the spectral reflectance measured for a cured product of the dental curable composition against a white background with a spectral colorimeter is substantially constant in a certain wavelength range.

Specifically, the present invention includes the following.

[1] A dental curable composition comprising a polymerizable monomer (A), a filler (B), a polymerization initiator (C), and a colorant (D), wherein the ratios $R_{650/600}$, $R_{700/600}$, and $R_{750/600}$ of spectral reflectances at 650 nm, 700 nm, and 750 nm wavelengths to a spectral reflectance at 600 nm wavelength all fall within a range of 97% to 103% when measured for a 1.0 mm-thick cured product of the dental curable composition against a white background with a spectral colorimeter.

[2] The dental curable composition according to [1], wherein a 1.0 mm-thick cured product of the dental curable composition satisfies a contrast ratio of 0.35 to 0.65 as defined by the following formula (1),

$$\text{Contrast ratio} = Y_b/Y_w \qquad (1),$$

where $Y_b$ represents a Y value of XYZ color system measured against a black background, and $Y_w$ represents a Y value of XYZ color system measured against a white background.

[3] The dental curable composition according to [1] or [2], wherein a 0.25 mm-thick cured product of the dental curable composition satisfies a light diffusivity LD of 0.0001 to 0.99 as defined by the following formula (2),

$$LD = (I_5/\cos 5°)/I_0 \qquad (2),$$

where I represents a luminous intensity of light transmitted through a cured product, and $I_0$ and $I_5$ represent luminous intensities of transmitted light at 0- and 5-degree angles, respectively, with respect to a direction perpendicular to a sample plate (a direction of incident light).

[4] The dental curable composition according to any one of [1] to [3], wherein a 1.0 mm-thick cured product of the dental curable composition has a chromaticity index a*/w of -3.0 to 2.0 as measured in L*a*b*color system with a standard whiteboard placed behind the cured product.

[5] The dental curable composition according to any one of [1] to [4], wherein the filler (B) comprises an inorganic fine particle (BF-1) having an average particle diameter of 0.05 to 1 $\mu$m.

[6] The dental curable composition according to any one of [1] to [5], wherein the filler (B) comprises an inorganic agglomerated particle (BF-2) formed by agglomeration of an inorganic primary particle (x), and the inorganic primary particle (x) has an average particle diameter of 0.001 to 1 $\mu$m.

[7] The dental curable composition according to [6], wherein the inorganic agglomerated particle (BF-2) comprises a light diffusive inorganic agglomerated particle (BF-2d), and the light diffusive inorganic agglomerated particle (BF-2d) has a refractive index that satisfies the following formula (3),

$$0.03 < |nP - nF_{BF\text{-}2d}| < 1.0 \qquad (3),$$

where nP represents a refractive index of a polymer obtained by polymerization of the polymerizable monomer (A), and $nF_{BF\text{-}2d}$ represents a refractive index of the light diffusive inorganic agglomerated particle (BF-2d).

[8] The dental curable composition according to any one of [1] to [7], wherein the filler (B) comprises an organic-inorganic composite filler (BC) containing an inorganic primary particle (x), and the inorganic primary particle (x) has an average particle diameter of 0.001 to 1 $\mu$m.

[9] The dental curable composition according to [8], wherein the organic-inorganic composite filler (BC) comprises a light diffusive organic-inorganic composite filler (BC-d), and the light diffusive organic-inorganic composite filler (BC-d) has a refractive index that satisfies the following formula (7),

$$0.03 < |nP - nF_{BC\text{-}d}| < 1.0 \qquad (7),$$

where nP represents a refractive index of a polymer obtained by polymerization of the polymerizable monomer (A), and $nF_{BC\text{-}d}$ represents a refractive index of the light diffusive organic-inorganic composite filler (BC-d).

Advantageous Effects of Invention

According to the present invention, a dental curable composition can be provided that enables a highly aesthetic restoration that blends in so well with the surroundings that it is almost indistinguishable from natural teeth, and that, despite being a single dental curable composition, shows good color compatibility with natural teeth over a wide range of shades. The present invention can also provide a dental curable composition that, despite being a single dental curable composition, shows good color compatibility with natural teeth over a wide range of shades, particularly in cases involving cavity floors in different classes of cavities, such as Class I, Class II, and Class V. A dental curable composition of the present invention can be suitably used for dental composite resins. According to the present invention, a dental curable composition can be provided whose cured product excels in ease of polishing and gloss retention. The present invention can also provide a dental curable composition that excels in ease of handling.

DESCRIPTION OF EMBODIMENTS

The present invention is described below in detail. A dental curable composition of the present invention is a dental curable composition comprising a polymerizable monomer (A), a filler (B), a polymerization initiator (C), and a colorant (D), wherein the ratios R of spectral reflectances at 650 nm, 700 nm, and 750 nm wavelengths to a spectral reflectance at 600 nm wavelength all fall within a range of 97% to 103% when measured for a 1.0 mm-thick cured product of the dental curable composition against a white background with a spectral colorimeter. With such a configuration, a highly aesthetic restoration can be achieved that blends in so well with the surroundings that it is almost indistinguishable from natural teeth, and the dental curable composition, despite being a single dental curable composition, shows good color compatibility with natural teeth over a wide range of shades, particularly in cases involving cavity floors in different classes of cavities, such as Class I, Class II, and Class V.

In a dental curable composition of the present invention, the ratios of spectral reflectances at 650 nm, 700 nm, and 750 nm wavelengths ($r_{650}$, $r_{700}$, and $r_{750}$, respectively) to a spectral reflectance at 600 nm wavelength ($r_{600}$) all fall within a range of 97.0% to 103.0% when measured for a 1.0 mm-thick cured product of the dental curable composition against a white background with a spectral colorimeter ($R_{650/600} = r_{650}/r_{600} \times 100$, $R_{700/600} = r_{700}/r_{600} \times 100$, $R_{750/600} = r_{750}/r_{600} \times 100$). Preferably, the ratios $R_{650/600}$, $R_{700/600}$, and $R_{750/600}$ all fall within a range of 97.5% to 102.5%, more preferably 97.8% to 102.2%, even more preferably 98.0% to 102.0%. With the spectral reflectance ratios confined in these ranges, the shade of a cavity floor of a natural tooth can more easily manifest in portions filled with the dental curable composition when it is used to fill cavities and restore natural teeth. With this effect, the dental curable composition, despite being a single dental curable composition, can provide a remarkably aesthetic restoration method for natural teeth over a wide range of shades, without preparing more than one kind of dental curable composition in different shades. In certain preferred embodiments, the ratios of spectral reflectances at 700 nm and 750 nm wavelengths ($r_{700}$ and $r_{750}$, respectively) to a spectral reflectance at 650 nm wavelength ($r_{650}$) both fall within a range of preferably 97.0% to 103.0%, more preferably 97.5% to 102.5%, even more preferably 97.8% to 102.2%, particularly preferably 98.0% to 102.0% when measured for a 1.0 mm-thick cured product of the dental curable composition against a white background with a spectral colorimeter ($R_{700/650} = r_{700}/r_{650} \times 100$, $R_{750/650} = r_{750}/r_{650} \times 100$).

The spectral reflectance ratios falling in these preferred ranges means that the spectral reflectances are essentially almost constant at 600 to 750 nm wavelengths, or having a curve that is almost plateau in this range. Here, the measurement system measures light (measurement light) that has transmitted through a cured product plate by being reflected at a white background following passage of incident light through the cured product plate. Having a spectral reflectance curve that is almost plateau in the foregoing range means that the measurement light is constant in this range. Here, the measurement light is constant when absorption of light by the cured product is constant for light reflected off the white background and passed through the cured product, irrespective of the wavelength, meaning that the background color can manifest directly on the surface. A cured product of a dental curable composition of the present invention shows reflection of light that is constant with respect to the yellow to red color of 600 to 750 nm wavelengths seen in the shade of natural teeth, and can very desirably manifest the background, or pick up the background color, in this color range. Clinically, these characteristics are particularly effective in cases involving bottom portions of cavities (cavity floors) classified according to the Black's classification, such as Class I, Class II, and Class V. The Black's classification refers to a system of categorizing cavities, and includes Class I, Class II, Class III, Class IV, and Class V. Class I cavities are those formed in caries originating in pits and fissures of molars, for example. Class II cavities are those originating in adjacent surfaces of molars. Class III cavities are those originating in adjacent surfaces of front teeth and canines, excluding the corners of incisors. Class IV cavities are those originating in adjacent surfaces of front teeth and canines, including the corners of incisors. Class V cavities are those occurring on the gingival one-third of teeth on the labial (cheek) or tongue (palate) side of the crown. When a dental curable composition of the present invention is used for restoration purposes in such cases, its cured product desirably manifests the shade of the cavity floors of natural teeth, and enables a highly aesthetic restoration that blends in well with the surroundings while the dental curable composition, despite being a single dental curable composition, shows good color compatibility with natural teeth over a wide range of shades.

The spectral reflectance can be measured with a spectral colorimeter that is capable of measuring reflected light. The spectral colorimeter used for measurement may be a commercially available product, for example, such as a spectral colorimeter SE7700 or SE6000 manufactured by Nippon Denshoku Industries Co., Ltd. The white background used for measurement is a standard whiteboard that has been calibrated so that the XYZ tristimulus values measured with a C/2 illuminant according to JIS Z 8722, 0°-45° methods satisfy $90 \leq X \leq 96$, $92 \leq Y \leq 98$, and $100 \leq Z \leq 116$. For measurement, the standard whiteboard is placed in close contact with a cured plate of the dental curable composition being measured. Specifically, the spectral reflectance of a cured product of a dental curable composition of the present invention can be measured using the method described in the EXAMPLES section below.

The spectral reflectance can be adjusted by the type of filler (B) or colorant (D) (described later), or by increasing or decreasing its proportion. Specifically, an uncolored curable composition is prepared from a polymerizable monomer (A), a filler (B), and a polymerization initiator (C), and the spectral reflectance is measured. Here, it is preferable that the spectral reflectance curve show a pattern of slight increase or a plateau in the 600 to 750 nm wavelength range. This can be controlled by the types or proportions of polymerizable monomer (A) and filler (B). The spectral reflectance curve can then be adjusted to have a plateau in this wavelength range by subsequently adding a colorant (D), particularly a yellow or red pigment.

A dental curable composition of the present invention shows good color compatibility with natural teeth even without strict control to reproduce the shade of natural teeth by bringing various shade-related parameters (for example, contrast ratio, chroma) closer to those of natural teeth. For the ability to further improve aesthetics, a 1.0 mm-thick cured product of a dental curable composition of the present invention has a contrast ratio of preferably 0.35 or more, more preferably 0.40 or more, even more preferably 0.43 or more, particularly preferably 0.45 or more. The contrast ratio is preferably 0.65 or less, more preferably 0.60 or less, even more preferably 0.57 or less, particularly preferably 0.55 or less. With the foregoing lower limits, it is possible to effectively reduce a dark dull impression in portions of teeth filled with the dental curable composition. With the foregoing upper limits, the shade of the cavity floor can effectively manifest in portions filled with the dental curable composition. In the present invention, a contrast ratio is a value determined based on the formula (1) below. In formula (1), $Y_b$ represents a Y value of XYZ color system measured against a black background, and $Y_w$ represents a Y value of XYZ color system measured against a white background, using a colorimeter.

$$\text{Contrast ratio} = Y_b/Y_w \qquad\qquad (1)$$

The contrast ratio is an index of transparency, indicating that the material is more opaque when the value is closer to 1, and is more transparent when the value is closer to 0.

The contrast ratio can be measured with a spectral colorimeter such as above, for example. The white background is a standard whiteboard, and is used for measurement by being brought into close contact with a cured plate of the dental curable composition being measured. A matte dark box is used as a black background. Specifically, the contrast ratio of a cured product of a dental curable composition of the present invention can be measured using the method described in the EXAMPLES section below.

The contrast ratio can be adjusted by adding a colorant (D) or by increasing or decreasing its proportion. Preferably, adjustments are made by varying the added amount of a white pigment, such as zinc white or titanium oxide, because it has a small impact on factors, for example, such as hue and chroma, in other colors.

Preferably, a dental curable composition of the present invention also has light diffusion properties. The light diffusion properties can be measured with a goniometer or goniophotometer. Specifically, the extent of light diffusion properties can be measured by determining the luminous intensity of diffuse transmitted light relative to specularly transmitted light after the light perpendicularly incident on a cured product of the dental curable composition is measured for the luminous intensity of specularly transmitted light, and the luminous intensity of diffuse transmitted light other than the specularly transmitted light. By having a certain degree of light diffusion properties, a dental curable composition of the present invention can produce the blurring effect that blurs the boundaries between natural teeth and the filled material when cavities in natural teeth are filled and restored with a dental curable composition of the present invention. With this effect, the shades of natural teeth and filled portions blend in, and the filled portions become unnoticeable, making it possible to provide a remarkably aesthetic restoration method. This is particularly effective in cases involving restoration of cavities extending from labial side to tongue side, such as in Class III or IV of the Black's classification. In such cases, the restored areas normally show an appearance with a dark, subdued color when a low-contrast-ratio (high-transparency) dental curable composition is used for restoration. Aesthetics can improve with the use of a dental curable composition having an increased contrast ratio (low transparency) provided by, for example, increasing the amount of a pigment such as titanium oxide. However, a dental curable composition imparted with light diffusion properties can provide a highly aesthetic natural appearance even with a relatively low contrast ratio (high transparency).

A 0.25 mm-thick cured product of a dental curable composition of the present invention has a light diffusivity LD of preferably 0.0001 or more, more preferably 0.001 or more, even more preferably 0.0015 or more as defined by the formula (2) below. The light diffusivity LD is preferably 0.99 or less, more preferably 0.97 or less, even more preferably 0.95 or less. Here, the light diffusivity LD is defined by the following formula (2).

$$LD = (I_5/\cos 5°)/I_0 \qquad (2),$$

where I represents a luminous intensity of light transmitted through a cured product, and $I_0$ and $I_5$ represent luminous intensities of transmitted light at 0- and 5-degree angles, respectively, with respect to a direction perpendicular to a sample plate (a direction of incident light).

In formula (2), the value of luminous intensity at 5° is divided by the cosine of this angle to convert it into a form that conforms to a measure perceivable by human eyes. That is, following the definition of illuminance, the luminous intensity can be converted into illuminance by dividing it by the cosine of the measurement angle. Accordingly, the light diffusion properties are stronger as the LD value approaches 1.

With the foregoing lower limits, it is possible to effectively reduce a dark dull impression in filled portions, or blur the boundaries between filled portions and natural teeth. With the foregoing upper limits, the shade of the cavity floor can effectively manifest in filled portions. The light diffusivity LD can be measured using the method described in the EXAMPLES section below.

The light diffusion properties can be adjusted by adding a light diffusive filler (described later), and by adjusting the amount added.

A dental curable composition of the present invention shows good color compatibility with natural teeth even without strict control to reproduce the shade of natural teeth by bringing various shade parameters (for example, contrast ratio, chroma) closer to those of natural teeth. However, aesthetics can further improve when a 1.0 mm-thick cured product of a dental curable composition of the present invention has an a*/w of preferably -3.0 or more, more preferably -2.5 or more, even more preferably -2.0 or more, particularly preferably -1.8 or more, where a*/w is a red chromaticity index in L*a*b*color system against a white background. Preferably, a*/w is 2.0 or less, more preferably 1.5 or less, even more preferably 1.0 or less, particularly preferably 0.5 or less. With these ranges of a*/w, the shade of the cavity floor can effectively manifest in filled portions.

The chromaticity index a*/w can be measured with a spectral colorimeter such as above. The white background is a standard whiteboard, and is used for measurement by being brought into close contact with a cured plate of the dental curable composition being measured. Specifically, the chromaticity index a*/w of a cured product of a dental curable composition of the present invention can be measured using the method described in the EXAMPLES section below.

The chromaticity index a*/w can be adjusted by adding a pigment, or by increasing or decreasing its proportion. Preferably, adjustments are made by varying the added amount of a red pigment, particularly, red iron oxide.

For the ability to further improve aesthetics, a 1.0 mm-thick cured product of a dental curable composition of the present invention has an L*/w of preferably 60.0 or more, more preferably 65.0 or more, even more preferably 68.0 or more, particularly preferably 70.0 or more in L*a*b*color system against a white background. Preferably, L*/w is 90.0 or less, more preferably 88.0 or less, even more preferably 85.0 or less, particularly preferably 82.0 or less.

For the ability to further improve aesthetics, a 1.0 mm-thick cured product of a dental curable composition of the present invention has a b*/w of preferably 0.0 or more, more preferably 1.0 or more, even more preferably 2.0 or more, particularly preferably 3.0 or more in L*a*b*color system against a white background. Preferably, b*/w is 50.0 or less, more preferably 45.0 or less, even more preferably 40.0 or less, particularly preferably 35.0 or less.

The following describes each component of a dental curable composition of the present invention.

[Polymerizable Monomer (A)]

A known polymerizable monomer used for dental curable compositions can be used as the polymerizable monomer (A) in a dental curable composition of the present invention. Particularly preferred for use are radical polymerizable monomers. Examples of such radical polymerizable monomers include esters of unsaturated carboxylic acids such as $\alpha$-cyanoacrylic acid, (meth)acrylic acid, $\alpha$-halogenated acrylic acid, crotonic acid, cinnamic acid, sorbic acid, maleic acid, and itaconic acid; (meth)acrylamide; (meth)acrylamide derivatives; vinyl esters; vinyl ethers; mono-N-vinyl derivatives; and styrene derivatives. The polymerizable monomer (A) may be used alone, or two or more thereof may be used in combination. Preferred among these are esters of unsaturated carboxylic acids, and (meth)acrylamide derivatives, more preferably (meth)acrylic acid esters, and (meth)acrylamide derivatives, even more preferably (meth)acrylic acid esters. The term "(meth)acryl" used in this specification is intended to be inclusive of both methacryl and acryl. As used herein, "(meth)acrylic monomer" is intended to be inclusive of both (meth)acrylic acid ester and (meth)acrylamide derivative. Examples of (meth)acrylic acid esters and (meth)acrylamide derivatives are as follows.

(i) Monofunctional (Meth)Acrylic Acid Esters and (Meth)Acrylamide Derivatives

Examples include methyl (meth)acrylate, isobutyl (meth)acrylate, benzyl (meth)acrylate, dodecyl (meth)acrylate, 2-(N,N-dimethylamino)ethyl (meth)acrylate, 2,3-dibromopropyl (meth)acrylate, 3-(meth)acryloyloxypropyltrimethoxysilane, 11-(meth)acryloyloxyundecyltrimethoxysilane, 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, phenoxyethylene glycol(meth)acrylate, isobornyl (meth)acrylate, 3-phenoxybenzyl (meth)acrylate, N-methylol(meth)acrylamide, N-hydroxyethyl(meth)acrylamide, N,N-bis(hydroxyethyl)(meth)acrylamide, N,N-dimethyl(meth)acrylamide, N,N-diethyl(meth)acrylamide, N,N-di-n-propyl(meth)acrylamide, N-ethyl-N-methyl(meth)acrylamide, (meth)acryloylmorpholine, (meth)acryloyloxydodecylpyridinium bromide, (meth)acryloyloxydodecylpyridinium chloride, (meth)acryloyloxyhexadecylpyridinium bromide, (meth)acryloyloxyhexadecylpyridinium chloride, ethoxylated-o-phenylphenol (meth)acrylate, ethoxylated-m-phenylphenol (meth)acrylate, ethoxylated-p-phenylphenol (meth)acrylate, propoxylated-o-phenylphenol (meth)acrylate, propoxylated-m-phenylphenol (meth)acrylate, propoxylated-p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, and 2-(p-phenoxyphenyl)ethyl (meth)acrylate. In view of good ease of handling of a paste of the dental curable composition obtained, and excellence of mechanical strength after cure, most preferred are ethoxylated-o-phenylphenol (meth)acrylate, and m-phenoxybenzyl (meth)acrylate.

(ii) Bifunctional (Meth)Acrylic Acid Esters

Examples include aromatic bifunctional (meth)acrylic acid esters, and aliphatic bifunctional (meth)acrylic acid esters.

Examples of the aromatic bifunctional (meth)acrylic acid esters include 2,2-bis((meth)acryloyloxyphenyl)propane, 2,2-bis[4-(3-acryloyloxy-2-hydroxypropoxy)phenyl]propane, 2,2-bis[4-(3-methacryloyloxy-2-hydroxypropoxy)phenyl]propane (commonly known as Bis-GMA), 2,2-bis(4-(meth)acryloyloxypolyethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, 2,2-bis(4-(meth)acryloyloxydipropoxyphenyl)propane, 2-(4-(meth)acryloyloxydipropoxyphenyl)-2-(4-(meth)acryloyloxytriethoxyphenyl)propane, 9,9-bis[4-(2-(meth)acryloyloxyethoxy)phenyl]fluorene, 9,9-bis[4-(2-(meth)acryloyloxypolyethoxy)phenyl]fluorene, diphenyl bis[3-(meth)acryloyloxypropyl]silane, and methylphenyl bis[3-(meth)acryloyloxypropyl]silane.

Examples of the aliphatic bifunctional (meth)acrylic acid esters include glycerol di(meth)acrylate, ethylene glycol di(meth)acrylate, diethylene glycol di(meth)acrylate, triethylene glycol diacrylate, triethylene glycol dimethacrylate (commonly known as 3G), propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylene bis(2-carbamoyloxyethyl)diacrylate, 2,2,4-trimethylhexamethylene bis(2-carbamoyloxyethyl)dimethacrylate (commonly known as UDMA), and dicyclohexyl bis[3-(meth)acryloyloxypropyl]silane.

In view of considerations such as improvement of the ease of handling of the dental curable composition, and improvement of the mechanical strength of the cured product obtained, more preferred as bifunctional (meth)acrylic acid esters are 2,2-bis[4-(3-acryloyloxy-2-hydroxypropoxy)phenyl]propane, 2,2-bis[4-(3-methacryloyloxy-2-hydroxypropoxy)phenyl]propane (Bis-GMA), 2,2-bis(4-(meth)acryloyloxypolyethoxyphenyl)propane (average number of moles of ethyleneoxy group added: 1 to 30), triethylene glycol diacrylate, triethylene glycol dimethacrylate (3G), 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylene bis(2-carbamoyloxyethyl)diacrylate, and 2,2,4-trimethylhexamethylene bis(2-carbamoyloxyethyl)dimethacrylate (UDMA), even more preferably 2,2-bis[4-(3-methacryloyloxy-2-hydroxypropoxy)phenyl]propane (Bis-GMA), 2,2-bis(4-methacryloyloxypolyethoxyphenyl)propane (average number of moles of ethyleneoxy group added: 1 to 30), triethylene glycol dimethacrylate (3G), 1,10-decanediol dimethacrylate, 1, 12-dodecanediol dimethacrylate, 1,2-bis(3-methacryloyloxy-2-hydroxypropyloxy)ethane, tricyclodecanedimethanol dimethacrylate, and 2,2,4-trimethylhexamethylene bis(2-carbamoyloxyethyl)dimethacrylate (UDMA), particularly preferably 2,2-bis[4-(3-methacryloyloxy-2-hydroxypropoxy)phenyl]propane (Bis-GMA), 2,2-bis(4-methacryloyloxypolyethoxyphenyl)propane (average number of moles of ethyleneoxy group added: 2.6), and triethylene glycol dimethacrylate (3G).

(iii) Tri- and Higher-Functional (Meth)Acrylic Acid Esters

Examples include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N'-(2,2,4-trimethylhexamethylene)bis[2-(aminocarboxy)propane-1,3-diol]tetra(meth)ac rylate, and 1,7-di(meth)acryloyloxy-2,2,6,6-tetra(meth)acryloyloxymethyl-4-oxaheptane.

Also preferred for use as polymerizable monomer (A) are oligomers and polymers having a radical polymerizable group such as a (meth)acrylic acid ester group. For example, it is possible to use polymerizable prepolymers described in JP S50-42696 A, unsaturated urethane-based oligomers described in JP 2011-144121 A, polyfunctional acrylate compounds described in JP 2006-510583 T, and prepolymers described in WO2020/122192.

It may be preferable that the polymerizable monomer (A) contain a functional monomer capable of imparting adhesive properties to adherends such as tooth structure, metals, and ceramics because a dental curable composition produced with such a functional monomer can show excellent adhesive properties to such materials, for example.

In view of providing excellent adhesive properties to tooth structure and base metals, the functional monomer may be, for example, a polymerizable monomer having a phosphoric acid group, such as 2-(meth)acryloyloxyethyl dihydrogen phosphate, 10-(meth)acryloyloxydecyl dihydrogen phosphate, and 2-(meth)acryloyloxyethylphenyl hydrogen phosphate; or a polymerizable monomer having a carboxylic acid group, such as 11-(meth)acryloyloxyundecane-1,1-dicarboxylic acid, and 4-(meth)acryloyloxyethoxycarbonylphthalic acid. In view of providing excellent adhesive properties to noble metals, the functional monomer may be, for example, 10-mercaptodecyl (meth)acrylate, 6-(4-vinylbenzyl-n-propyl)amino-1,3,5-triazine-2,4-dithione, a thiouracil derivative described in JP H10-1473 A, or a compound having a sulfur element described in JP H11-92461 A. In view of effective adhesion to ceramics, porcelain, and other dental curable compositions, the functional monomer may be, for example, a silane coupling agent such as $\gamma$-(meth)acryloyloxypropyltrimethoxysilane.

The content of the polymerizable monomer (A) in a dental curable composition of the present invention is not particularly limited. However, in view of properties such as the ease of handling of the dental curable composition obtained, and the mechanical strength of the cured product, the content of polymerizable monomer (A) is preferably 1 mass% or more, more preferably 2 mass% or more, even more preferably 5 mass% or more, or may be 8 mass% or more, or 15 mass% or more, based on the total mass of the dental curable composition. The content of polymerizable monomer (A) is preferably 70 mass% or less, more preferably 50 mass% or less, even more preferably 40 mass% or less, particularly preferably 30 mass% or less based on the total mass of the dental curable composition.

[Filler (B)]

A dental curable composition of the present invention comprises a filler (B).

The overall particle shape of filler (B) is not particularly limited, and the filler (B) may be used in the form of an irregularly shaped powder or a spherical powder. With an irregularly shaped filler (B), a dental curable composition can be obtained that particularly excels in the mechanical strength, gloss retention, and abrasion resistance of the cured product. With a spherical filler (B), a dental curable composition can be provided that has smooth, easy-to-stretch paste properties with excellent ease of handling. Irregularly shaped fillers provide higher mechanical strength than spherical fillers probably because the particles of irregularly shaped fillers bite into one another in the cured product. Irregularly shaped fillers provide higher gloss retention and abrasion resistance than spherical fillers probably because the particles of irregularly shaped fillers do not easily detach themselves from the surface of the cured product, and prevent the cured product from having a rougher surface due to an increase of marks created by detached particles. Spherical fillers are easier to handle than irregularly shaped fillers probably because of the smaller specific surface area and smaller areas of contact with polymerizable monomer (A). The overall shape of filler (B) can be appropriately selected according to intended use of the dental curable composition. In view of excellence of mechanical strength and gloss retention, it is preferable to use irregularly shaped filler (B).

The filler (B) in the present invention can be broadly classified into inorganic filler (BF), organic-inorganic composite filler (BC), and organic filler (BP).

Examples of the inorganic filler (BF) include various types of glasses [preferably those containing silica as a main component, and, optionally, an oxide of heavy metal, boron, aluminum, or the like (examples includes glass powders of common compositions, such as fused silica, quartz, soda-lime-silica glass, E glass, C glass, and borosilicate glass (PYREX® glass); and glass powders for dentistry, such as barium glass (GM27884, 8235 manufactured by Schott, and E-2000, E-3000 manufactured by ESSTECH), strontium borosilicate glass (E-4000 manufactured by ESSTECH), lanthanum glass-ceramic (GM31684 manufactured by Schott), and fluoroaluminosilicate glass (GM35429, G018-091, G018-117 manufactured by Schott))], various types of ceramics, alumina, composite oxides (such as silica-titania, silica-zirconia, and silica-ytterbia), diatomaceous earth, kaolin, clay minerals (such as montmorillonite), activated earth, synthetic zeolite, mica, calcium fluoride, ytterbium fluoride, yttrium fluoride, calcium phosphate, barium sulfate, zirconium oxide, titanium oxide, and hydroxyapatite. These may be used alone, or two or more thereof may be used in combination. Preferred are those containing silica as a main component (containing 5 mass% or more of silica, preferably 10 mass% or more of silica). Preferably, it is preferable to comprise an inorganic filler (such as barium glass, alumina, silica-titania, silica-zirconia, and silica-ytterbia) containing metallic elements having high radiopacity (such as barium, zirconium, aluminum, and ytterbium), or ytterbium fluoride.

The inorganic filler (BF) may be amorphous or crystalline, or may be a mixture of both. It is, however, preferable that the inorganic filler (BF) include at least an amorphous portion. Transparency improves as the proportion of amorphous portions in the inorganic filler (BF) increases.

The inorganic filler (BF) in the present invention preferably comprises an inorganic fine particle (BF-1) having an average particle diameter of 0.05 to 1 $\mu$m, more preferably 0.08 to 0.9 $\mu$m , even more preferably 0.1 to 0.8 $\mu$m. With these lower limits of the average particle diameter of inorganic fine particle (BF-1), it is possible to more effectively reduce stickiness or stringiness in the dental curable composition obtained, and ease of handling improves. With the foregoing upper limits of the average particle diameter of inorganic fine particle (BF-1), ease of polishing and gloss retention improve in a cured product of the dental curable composition obtained. Because it is easier to provide glossiness comparable to that of natural teeth, it is also possible to provide a filling restoration that is highly color compatible.

The average particle diameter of the inorganic fine particle (BF-1) can be determined by a laser diffraction scattering method, or electron microscopy of particles. For example, the average particle diameter can be measured by volume by a laser diffraction scattering method with a laser diffraction particle size distribution analyzer (e.g., SALD-2300 manufactured by Shimadzu Corporation), using ethanol or a 0.2% sodium hexametaphosphate aqueous solution as dispersion medium. A laser diffraction scattering method is convenient for the measurement of particles having a particle diameter of 0.1 $\mu$m or more. For electron microscopy, a scanning electron microscope (e.g., SU3500, SU3800, or S-4000 manufactured by Hitachi High-Technologies Corporation) can be used. As a specific example of electron microscopy, particles may be photographed with an electron microscope, and the size of particles (at least 200 particles) observed in a unit field of the micrograph may be measured using image-analyzing particle-size-distribution measurement software (Macview manufactured by Mountech Co., Ltd.). Here, the particle diameter is determined as an arithmetic mean value of the maximum and minimum lengths of particles, and the average particle diameter is calculated from the number of particles and the particle diameter. More specifically, the average particle diameter of inorganic fine particle (BF-1) can be measured using the method described in the EXAMPLES section. In this specification, the average particle diameter of inorganic filler (BF) means an average particle diameter before surface treatment when the inorganic filler (BF) is surface treated.

The refractive index of inorganic fine particle (BF-1) is not particularly limited. However, it is easier to increase the transparency of the cured product obtained when the refractive index of inorganic fine particle (BF-1) is brought closer to the refractive indices of components other than the inorganic fine particle (BF-1) in the cured product of the dental curable composition. To this end, the refractive index of inorganic fine particle (BF-1) is preferably 1.40 or more, more preferably 1.45 or more, even more preferably 1.50 or more, and is preferably 1.63 or less, more preferably 1.60 or less, even more preferably 1.58 or less. The refractive index of inorganic fine particle (BF-1) can be controlled by, for example, adjusting the type and proportion of the metallic elements contained in the inorganic fine particle (BF-1).

Preferably, the inorganic filler (BF) in a dental curable composition of the present invention comprises an inorganic agglomerated particle (BF-2) formed by agglomeration of inorganic primary particle (x). The inorganic primary particle (x) has an average particle diameter of preferably 0.001 to 1 $\mu$m , more preferably 0.005 to 0.8 $\mu$m , even more preferably 0.01 to 0.5 $\mu$m. With these lower limits of the average particle diameter of inorganic primary particle (x), the inorganic agglomerated particle (BF-2) can be prevented from having an unnecessarily large specific surface area, and it is possible to improve ease of handling by more effectively reducing stickiness or stringiness in the dental curable composition. With the foregoing upper limits of the average particle diameter of inorganic primary particle (x), ease of polishing and gloss retention improve in a cured product of the dental curable composition obtained. Because it is easier to provide glossiness comparable to that of natural teeth, it is also possible to provide a filling restoration that is highly color compatible. The average particle diameter of inorganic primary particle (x) can be determined by the laser diffraction scattering method or electron microscopy described above.

In the present invention, the inorganic agglomerated particle (BF-2) has a form of an agglomerated particle formed by agglomeration of inorganic primary particle (x). Commercially available inorganic fillers typically exist in the form of aggregates. The cohesion of commercially available inorganic fillers is so weak that these fillers break into the particle size indicated by the manufacturer when 10 mg of its powder is added and ultrasonically dispersed at 40 W and 39 KHz for 30 minutes in 300 mL of a dispersion medium such as water, 5 mass% or less of a surfactant (e.g., sodium hexametaphosphate) in water, or ethanol. In contrast, the inorganic agglomerated particles (BF-2) of the present invention are strongly held together, and become hardly dispersed even under these conditions.

In a preferred method of preparing a strong agglomerate of particles from an aggregate of commercially available inorganic fillers, the inorganic filler is heated to a temperature range just below the temperature that melts the inorganic filler so that the adjoining inorganic filler particles, under the applied heat, lightly fuse together and increase cohesion. Here, the inorganic filler may have a form of an aggregate before heating, in order to control the shape of the agglomerated particle. An aggregate can be formed, for example, by applying pressure to the inorganic filler placed in a suitable container, or by dispersing the inorganic filler in a solvent, and removing the solvent using a method such as spray drying.

In another preferred method of preparing the inorganic agglomerated particle (BF-2) strongly held together by inorganic filler particles, a sol such as a silica sol, an alumina sol, a titania sol, or a zirconia sol prepared by a wet method is dried using a method such as freeze drying or spray drying, and optionally subjected to a heat treatment. In this way, the inorganic agglomerated particle (BF-2) can be obtained with ease that is strongly held together by particles. Specific examples of the sols include fine spherical silica particles (Seahostar® manufactured by Nippon Shokubai Co., Ltd. under this trade name; e.g., KE series, a surface-treated type), a silica organosol (OSCAL® manufactured by JGC C & C under this trade name), a titania sol (QUEEN TITANIC series manufactured by Nissan Chemical Corporation under this trade name), a silica sol (SNOWTEX® manufactured by Nissan Chemical Corporation under this trade name), an alumina sol (Aluminasol-100, Aluminasol-200, Aluminasol-520 manufactured by Nissan Chemical Corporation under these trade names), and a zirconia sol (NanoUse® ZR series manufactured by Nissan Chemical Corporation under this trade name). The shape of the inorganic agglomerated particle (BF-2) is not particularly limited, and may be appropriately selected for use.

The heat treatment conditions in the method of production of inorganic agglomerated particle (BF-2) cannot be generalized as a rule because the optimum conditions (temperature, time) depend on factors such as the composition of the inorganic primary particle (x). For many compositions, however, the preferred heat treatment temperature (firing temperature) ranges from 500 to 1,200°C. An overly low heat treatment temperature tends to cause a decrease of mechanical strength in a cured product of the dental curable composition finally obtained. An overly high heat treatment temperature causes excessive fusing between inorganic primary particles (x), and this tends to impair the polishability and gloss retention of the cured product obtained from the final dental curable composition.

More detailed processing conditions for the heat treatment can be determined by, for example, choosing conditions with which no crystalline structure can be confirmed in a powder X-ray diffraction analysis of inorganic agglomerated particles (BF-2) produced as secondary particles (agglomerated particles) under several firing conditions in the foregoing heat-treatment temperature ranges. As another example, dental curable compositions may be produced from inorganic agglomerated particles (BF-2) produced in the manner described above, and the heat treatment conditions may be decided after measuring properties such as the flexural strength of cured products formed from these dental curable compositions, or gloss on polished surfaces of the cured products. In many cases, an insufficient heat treatment often leads to a cured product with insufficient flexural strength. When overly heat treated, the resultant cured product tends to have a low gloss on polished surfaces, in addition to showing an unnaturally opaque appearance. This is probably because an over heat treatment causes crystallization to occur in parts of the constituent components, increasing the refractive index of the inorganic agglomerated particle (BF-2), and producing an unnaturally white color, different from the whiteness of natural teeth, in a cured product formed by the dental curable composition using such an inorganic agglomerated particle (BF-2). An over heat treatment also increases the hardness of inorganic agglomerated particle (BF-2), and this appears to impair polishability by making it difficult to grind a cured product formed by the dental curable composition.

In the present invention, the inorganic agglomerated particle (BF-2) has a specific surface area of preferably 10 $m^2$/g or more, preferably 15 $m^2$/g or more, more preferably 18 $m^2$/g or more, even more preferably 20 $m^2$/g or more. The inorganic agglomerated particle (BF-2) has a specific surface area of 300 $m^2$/g or less, preferably 250 $m^2$/g or less, more preferably 200 $m^2$/g or less, even more preferably 190 $m^2$/g or less. The inorganic agglomerated particle (BF-2) may have a specific surface area of 170 $m^2$/g or less, or 150 $m^2$/g or less. With the foregoing lower limits of the specific surface area of inorganic agglomerated particle (BF-2), a cured product of the dental curable composition obtained can have improved ease of polishing, and improved color compatibility. With the foregoing upper limits of the specific surface area of inorganic agglomerated particle (BF-2), the inorganic agglomerated particle (BF-2) can be added in increased amounts, and the mechanical strength improves in the cured product obtained. The specific surface area of inorganic agglomerated particle (BF-2) means a specific surface area of the agglomerated particle (secondary particle).

The specific surface area of inorganic agglomerated particle (BF-2) can be determined by the BET method. Specifically, the specific surface area of inorganic agglomerated particle (BF-2) can be measured using, for example, a specific surface area measurement device (e.g., BELSORP-mini series manufactured by MicrotracBEL Corp.). More specifically, the specific surface area of inorganic agglomerated particle (BF-2) can be measured using the method described in the EXAMPLES section below.

The inorganic agglomerated particle (BF-2) in the present invention has an average particle diameter of preferably 0.5 $\mu$m or more, more preferably 1 $\mu$m or more, even more preferably 1.5 $\mu$m or more, particularly preferably 2 $\mu$m or more. The inorganic agglomerated particle (BF-2) has an average particle diameter of preferably 50 $\mu$m or less, more preferably 40 $\mu$m or less, even more preferably 30 $\mu$m or less, particularly preferably 20 $\mu$m or less. With the foregoing lower limits of the average particle diameter of inorganic agglomerated particle (BF-2), it is possible to more effectively reduce stickiness or stringiness in the dental curable composition obtained, and the ease of handling improves. With the foregoing upper limits of the average particle diameter of inorganic agglomerated particle (BF-2), it is possible to more effectively reduce roughness or runniness in the dental curable composition obtained, and the ease of handling improves. The average particle diameter of inorganic agglomerated particle (BF-2) means an average particle diameter of the agglomerated particle (secondary particle). The average particle diameter of inorganic agglomerated particle (BF-2) can be determined by the laser diffraction scattering method or electron microscopy described above. The average particle diameter of inorganic agglomerated particle (BF-2) can be measured using the same method used for the measurement of inorganic fine particle (BF-1), specifically, by the method described in the EXAMPLES section below.

Preferably, the inorganic agglomerated particle (BF-2) in a dental curable composition of the present invention comprises a light diffusive inorganic agglomerated particle (BF-2d). The light diffusive inorganic agglomerated particle (BF-2d) is formed by agglomeration of inorganic primary particle (x), as in the inorganic agglomerated particle (BF-2) described above. The light diffusive inorganic agglomerated particle (BF-2d) means a particle with a refractive index satisfying the formula (3) below, preferably the formula (4), more preferably the formula (5) below, even more preferably the formula (6) below. The refractive index of light diffusive inorganic agglomerated particle (BF-2d) can be measured by the method described in the EXAMPLES section below.

$$0.03 < |nP - nF_{BF-2d}| < 1.0 \qquad (3)$$

$$0.04 < |nP - nF_{BF-2d}| < 0.6 \qquad (4)$$

$$0.05 < |nP - nF_{BF-2d}| < 0.4 \qquad (5)$$

$$0.06 < |nP - nF_{BF-2d}| < 0.2 \qquad (6),$$

where nP represents a refractive index of a polymer obtained by polymerization of the polymerizable monomer (A), and $nF_{BF-2d}$ represents a refractive index of the light diffusive inorganic agglomerated particle (BF-2d).

With the foregoing lower limit of the refractive index of light diffusive inorganic agglomerated particle (BF-2d) defined by formula (3), sufficient light diffusion properties can be imparted to the dental curable composition obtained. By imparting sufficient light diffusion properties, the dental curable composition obtained can provide a remarkably aesthetic restoration method by blurring the margin (boundary) between natural teeth and the filled material, and making the filled portion unnoticeable when used to restore cavities in natural teeth. With the foregoing upper limit defined by formula (3), certain transparency can be imparted to the dental curable composition obtained. By imparting certain transparency, the shade of a cavity floor in natural teeth can manifest in the filled portion when the dental curable composition obtained is used to restore cavities in natural teeth. With these effects, a single dental curable composition can provide a remarkably aesthetic restoration method for natural teeth over a wide range of shades, without preparing more than one kind of dental curable composition of different shades.

In the present invention, the refractive index nP of a polymer obtained by polymerization of polymerizable monomer (A) means a value measured with an abbe refractometer for a 1.0 mm-thick polymer obtained after a mixture prepared by mixing all the components (e.g., polymerization initiator (C)) except for filler (B) and colorant (D) into polymerizable monomer (A) is subjected to cast polymerization under predetermined conditions, as will be described in the EXAMPLES section below. When the polymerizable monomer (A) is a single polymerizable monomer, the refractive index nP is the refractive index of a mixture containing a homopolymer of the polymerizable monomer. When the polymerizable monomer (A) contains more than one kind of polymerizable monomer, the refractive index nP is the refractive index of a mixture containing a random copolymer of these polymerizable monomers. Here, "refractive index" means a refractive index at 25°C, unless otherwise specifically stated.

The inorganic primary particle (x) in the light diffusive inorganic agglomerated particle (BF-2d) may use any of the raw materials given above as being usable for the inorganic filler (BF). However, in order to control the refractive index to satisfy the foregoing formulae, particularly preferred for use are various types of glass powders of common compositions (such as fused silica, quartz, soda-lime-silica glass, E glass, C glass, and borosilicate glass (PYREX® glass)), ceramics, alumina, and composite oxides such as silica-titania, silica-zirconia, and silica-ytterbia. These may be used alone, or two or more thereof may be used in combination.

Preferably, the light diffusive inorganic agglomerated particle (BF-2d) may have an average particle diameter selected from the range of average particle diameters given above as being preferable for the inorganic agglomerated particle (BF-2). With the foregoing lower limits of the average particle diameter of light diffusive inorganic agglomerated particle (BF-2d), it is possible to impart white light diffusion properties similar to those seen in natural teeth, in addition to more effectively reducing stickiness or stringiness in the dental curable composition obtained, and improving the ease of handling. When the average particle diameter of light diffusive inorganic agglomerated particle (BF-2d) is below the foregoing lower limits, the scattered light appears more blue in tint, and the color compatibility with natural teeth decreases. With the foregoing upper limits of the average particle diameter of light diffusive inorganic agglomerated particle (BF-2d), it is possible to more effectively reduce roughness or runniness in the dental curable composition obtained, and the ease of handling improves.

The inorganic agglomerated particle (BF-2) may comprise an inorganic agglomerated particle (BF-2e) other than the light diffusive inorganic agglomerated particle (BF-2d) (hereinafter, also referred to simply as "additional inorganic agglomerated particle (BF-2e)"). Preferably, the additional inorganic agglomerated particle (BF-2e) may have an average particle diameter selected from the range of average particle diameters given above as being preferable for the inorganic agglomerated particle (BF-2), as with the case with the light diffusive inorganic agglomerated particle (BF-2d). The additional inorganic agglomerated particle (BF-2e) can be produced by using the same method described for inorganic agglomerated particle (BF-2). The inorganic primary particle (x) in the additional inorganic agglomerated particle (BF-2e) may use any of the raw materials given above as being usable for the inorganic filler (BF).

The inorganic filler (BF) may comprise an inorganic particle (BF-3) other than the inorganic fine particle (BF-1) and inorganic agglomerated particle (BF-2) (hereinafter, also referred to simply as "additional inorganic particle (BF-3)"). The additional inorganic particle (BF-3) has an average particle diameter of preferably more than 1 $\mu$m and 30 $\mu$m or less, more preferably more than 1 $\mu$m and 20 $\mu$m or less, even more preferably more than 1 $\mu$m and 10 $\mu$m or less. The average particle diameter of additional inorganic particle (BF-3) can be determined by a laser diffraction scattering method, or electron microscopy of particles. For example, the average particle diameter can be measured by volume by a laser diffraction scattering method with a laser diffraction particle size distribution analyzer (e.g., SALD-2300 manufactured by Shimadzu Corporation), using ethanol or a 0.2% sodium hexametaphosphate aqueous solution as dispersion medium.

The organic-inorganic composite filler (BC) in the present invention refers to a filler comprising the inorganic filler (BF) and a polymer of a polymerizable monomer (A').

The organic-inorganic composite filler (BC) has an average particle diameter of preferably 0.5 $\mu$m or more, more preferably 1 $\mu$m or more, even more preferably 1.5 $\mu$m or more, particularly preferably 2 $\mu$m or more. The organic-inorganic composite filler (BC) has an average particle diameter of preferably 50 $\mu$m or less, more preferably 40 $\mu$m or less, even more preferably 30 $\mu$m or less, particularly preferably 20 $\mu$m or less. With the foregoing lower limits of the average particle diameter of organic-inorganic composite filler (BC), it is possible to more effectively reduce stickiness or stringiness in the dental curable composition obtained, and the ease of handling improves. With the foregoing upper limits of the average particle diameter of organic-inorganic composite filler (BC), it is possible to more effectively reduce roughness or runniness in the dental curable composition obtained, and the ease of handling improves. The average particle diameter of organic-inorganic composite filler (BC) can be determined by the laser diffraction scattering method or electron microscopy described above.

The method of preparation of the organic-inorganic composite filler (BC) in the present invention is not particularly limited. For example, the organic-inorganic composite filler (BC) can be prepared by adding a polymerizable monomer (A') and a known polymerization initiator to the inorganic filler (BF) to prepare a paste-like mixture, and pulverizing this mixture after polymerization by solution polymerization, suspension polymerization, emulsion polymerization, or bulk polymerization.

The content of the inorganic filler (BF) in organic-inorganic composite filler (BC) is preferably 10 parts or more by mass, more preferably 20 parts or more by mass, even more preferably 30 parts or more by mass, particularly preferably 40 parts or more by mass relative to total 100 parts by mass of the inorganic filler (BF) and polymerizable monomer (A') in the organic-inorganic composite filler (BC). With these lower limits, the mechanical strength improves in the dental curable composition obtained. The upper limits are not particularly limited. However, because the paste viscosity increases for reasons related to production, the upper limit is preferably 99 parts or less by mass, more preferably 95 parts or less by mass.

The polymerizable monomer (A') is preferably any of the polymerizable monomers given above as examples that can be used as polymerizable monomer (A) in a dental curable composition of the present invention. In these examples, the absolute value of the difference between the refractive index of the polymerizable monomer (A') upon cure, and the refractive index of the inorganic filler (BF) in the organic-inorganic composite filler (BC) is preferably 0.30 or less, more preferably 0.20 or less. With these upper limits, the transparency of the organic-inorganic composite filler (BC) itself improves, and a highly aesthetic dental curable composition can be provided.

In view of considerations such as ease of polishing, the inorganic primary particle (x) is preferably used as the inorganic filler (BF) in the organic-inorganic composite filler (BC).

Preferably, the organic-inorganic composite filler (BC) in a dental curable composition of the present invention comprises a light diffusive organic-inorganic composite filler (BC-d). The light diffusive organic-inorganic composite filler (BC-d) comprises the inorganic filler (BF) and a polymer of a polymerizable monomer (A'), as does the organic-inorganic composite filler (BC). The light diffusive organic-inorganic composite filler (BC-d) means a filler with a refractive index satisfying the formula (7) below, preferably the formula (8) below, more preferably the formula (9) below, even more preferably the formula (10) below. The refractive index of light diffusive organic-inorganic composite filler (BC-d) can be measured using the method described in the EXAMPLES section below.

$$0.03 < |nP - nF_{BC-d}| < 1.0 \qquad (7)$$

$$0.04 < |nP - nF_{BC-d}| < 0.6 \qquad (8)$$

$$0.05 < |nP - nF_{BC-d}| < 0.4 \qquad (9)$$

$$0.06 < |nP - nF_{BC-d}| < 0.2 \qquad (10),$$

where $nP$ represents a refractive index of a polymer obtained by polymerization of the polymerizable monomer (A), and $nF_{BC-d}$ represents a refractive index of the light diffusive organic-inorganic composite filler (BC-d).

With the foregoing lower limits of the refractive index of light diffusive organic-inorganic composite filler (BC-d) defined by these formulae, sufficient light diffusion properties can be imparted to the dental curable composition obtained. By imparting sufficient light diffusion properties, the dental curable composition obtained can provide a remarkably aesthetic restoration method by blurring the margin (boundary) between natural teeth and the filled material, and making the filled portion unnoticeable when used to restore cavities in natural teeth. With the foregoing upper limits defined by the formulae above, certain transparency can be imparted to the dental curable composition obtained. By imparting certain transparency, the shade of a cavity floor in natural teeth can manifest in the filled portion when the dental curable composition obtained is used to restore cavities in natural teeth. With these effects, a single dental curable composition can provide a remarkably aesthetic restoration method for natural teeth over a wide range of shades, without preparing more than one kind of dental curable composition of different shades.

The inorganic primary particle (x) can preferably be used as the inorganic filler (BF) in the light diffusive organic-inorganic composite filler (BC-d). The inorganic primary particle (x) may use any of the raw materials given above as being usable for the inorganic filler (BF). However, in order to control the refractive index to satisfy the foregoing formulae, particularly preferred for use are various types of glass powders of common compositions (such as fused silica, quartz, soda-lime-silica glass, E glass, C glass, and borosilicate glass (PYREX® glass)), ceramics, alumina, and composite oxides such as silica-titania, silica-zirconia, and silica-ytterbia. These may be used alone, or two or more thereof may be used in combination.

Preferably, the light diffusive organic-inorganic composite filler (BC-d) may have an average particle diameter selected from the range of average particle diameters given above as being preferable for the inorganic agglomerated particle (BF-2). With the foregoing lower limits of the average particle diameter of light diffusive organic-inorganic composite filler (BC-d), it is possible to impart white light diffusion properties similar to those seen in natural teeth, in addition to more effectively reducing stickiness or stringiness in the dental curable composition obtained, and improving the ease of handling. When the average particle diameter of light diffusive organic-inorganic composite filler (BC-d) is below the foregoing lower limits, the scattered light appears more blue in tint, and the color compatibility with natural teeth decreases. With the foregoing upper limits of the average particle diameter of light diffusive organic-inorganic composite filler (BC-d), it is possible to more effectively reduce roughness or runniness in the dental curable composition obtained, and the ease of handling improves. The average particle diameter of light diffusive organic-inorganic composite filler (BC-d) can be measured using the same method used for the measurement of inorganic fine particle (BF-1), as with the case of the average particle diameter of inorganic agglomerated particle (BF-2). Specifically, the average particle diameter of light diffusive organic-inorganic composite filler (BC-d) can be measured using the method described in the EXAMPLES section below.

A surface treatment is not required for inorganic filler (BF) and organic-inorganic composite filler (BC). It is, however, preferable that the inorganic filler (BF) and organic-inorganic composite filler (BC) be surface-treated because surface treatment hydrophobizes the surfaces and improves the affinity for polymerizable monomer (A), and allows the inorganic filler (BF) and organic-inorganic composite filler (BC) to be contained in increased amounts. A surface treatment agent can be used for surface treatment. The type of surface treatment agent is not particularly limited, and a known surface treatment agent can be used, for example, such as a silane coupling agent, an organotitanium coupling agent, an organozirconium coupling agent, or an organoaluminum coupling agent. The surface treatment agent may be used alone, or two or more thereof may be used in combination. A silane coupling agent is preferred in view of considerations such as the affinity of inorganic filler (BF) and organic-inorganic composite filler (BC) for polymerizable monomer (A), and availability.

The silane coupling agent is preferably a compound represented by the following general formula (11), though the type of silane coupling agent is not particularly limited.

$$H_2C=CR^4-CO-R^5-(CH_2)_q-SiR^6_pR^7_{(3-p)} \qquad (11)$$

, wherein $R^4$ is a hydrogen atom or a methyl group, $R^5$ is an oxygen atom, a sulfur atom, or $-NR^8-$, where $R^8$ is a hydrogen atom or a C1 to C8 aliphatic group (may be linear, branched, or cyclic), $R^6$ is a hydrolyzable group, $R^7$ is a C1 to C6 hydrocarbon group, p is an integer of 1 to 3, q is an integer of 1 to 13, and the plurality of $R^6$ and $R^7$ each may be the same or different.

In the general formula (11), $R^4$ is a hydrogen atom or a methyl group, preferably a methyl group. $R^5$ is an oxygen atom, a sulfur atom, or $-NR^8-$, preferably an oxygen atom. $R^8$ represents a hydrogen atom, or a C1 to C8 aliphatic group (may be linear, branched, or cyclic), and the C1 to C8 aliphatic group represented by $R^8$ may be a saturated aliphatic group (such as an alkyl group or a cycloalkylene group (such as a cyclohexyl group)), or an unsaturated aliphatic group (such as an alkenyl group, or an alkynyl group). In view of considerations such as availability, ease of production, and chemical stability, preferred are saturated aliphatic groups, more preferably alkyl groups. Examples of the alkyl groups include methyl, ethyl, n-propyl, isopropyl, n-butyl, isobutyl, sec-butyl, tert-butyl, n-pentyl, isopentyl, n-hexyl, n-heptyl, 2-methylhexyl, and n-octyl. Preferably, $R^8$ is a hydrogen atom or a C1 to C4 alkyl group, more preferably a hydrogen atom or a C1 to C3 alkyl group, even more preferably a hydrogen atom.

In the general formula (11), examples of the hydrolyzable group represented by $R^6$ include alkoxy groups such as a methoxy group, an ethoxy group, and a butoxy group; halogen atoms such as a chlorine atom and a bromine atom; and an isocyanate group. When a plurality of $R^6$ exists, $R^6$ may be the same or different from one another. Preferably, $R^6$ is an alkoxy group, more preferably a methoxy group or an ethoxy group, even more preferably a methoxy group.

In the general formula (11), examples of the C1 to C6 hydrocarbon group represented by $R^7$ include a C1 to C6 alkyl group (may be cyclic), a C2 to C6 alkenyl group (may be cyclic), and a C2 to C6 alkynyl group. When a plurality of $R^7$ exists, $R^7$ may be the same or different from one another.

Examples of the C1 to C6 alkyl group include methyl, ethyl, n-propyl, isopropyl, n-butyl, isobutyl, sec-butyl, tert-butyl, n-pentyl, isopentyl, neopentyl, tert-pentyl, n-hexyl, cyclopropyl, cyclobutyl, cyclopentyl, and cyclohexyl.

Examples of the C2 to C6 alkenyl group include vinyl, allyl, 1-methylvinyl, 1-propenyl, butenyl, pentenyl, hexenyl, cyclopropenyl, cyclobutenyl, cyclopentenyl, and cyclohexenyl.

Examples of the C2 to C6 alkynyl group include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, and 1-ethyl-3-butynyl.

In the general formula (11), p is an integer of 1 to 3, preferably 2 or 3, more preferably 3. In the general formula (11), q is an integer of 1 to 13, preferably an integer of 2 to 12, more preferably an integer of 3 to 11.

Specific examples of the silane coupling agent represented by the general formula (11) include methacryloyloxymethyltrimethoxysilane, 2-methacryloyloxyethyltrimethoxysilane, 3-methacryloyloxypropyltrimethoxysilane, 4-methacryloyloxybutyltrimethoxysilane, 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane, 9-methacryloyloxynonyltrimethoxysilane, 10-methacryloyloxydecyltrimethoxysilane, 11-methacryloyloxyundecyltrimethoxysilane, 12-methacryloyloxydodecyltrimethoxysilane, 13-methacryloyloxytridecyltrimethoxysilane, 11-methacryloyloxyundecyldichloromethylsilane, 11-methacryloyloxyundecyltrichlorosilane, and 12-methacryloyloxydodecyldimethoxymethylsilane. The silane coupling agent may be used alone, or two or more thereof may be used in combination. In view of availability, preferred among these is 3-methacryloyloxypropyltrimethoxysilane. In view of further improvement of the affinity of inorganic filler (BF) and organic-inorganic composite filler (BC) for polymerizable monomer (A), preferred are 8-methacryloyloxyoctyltrimethoxysilane, 9-methacryloyloxynonyltrimethoxysilane, 10-methacryloyloxydecyltrimethoxysilane, and 11-methacryloyloxyundecyltrimethoxysilane.

The surface treatment method is not particularly limited, and may be a known method.

The surface treatment agent may be used in any amount. For example, 1 part or more by mass of surface treatment agent may be used relative to 100 parts by mass of the filler before surface treatment. The amount of surface treatment agent is preferably 5 parts or more by mass, more preferably 6 parts or more by mass, even more preferably 8 parts or more by mass, particularly preferably 10 parts or more by mass, most preferably 20 parts or more by mass, and is preferably 50 parts or less by mass, more preferably 45 parts or less by mass, even more preferably, 40 parts or less by mass, relative to 100 parts by mass of the filler before surface treatment. With the foregoing lower limits of the amount of surface treatment agent, the mechanical strength can more easily improves in the cured product obtained. With the foregoing upper limits of the amount of surface treatment agent, it is possible to reduce a decrease in the mechanical strength of the cured product due to the excess surface treatment agent.

Examples of the organic filler (BP) in the present invention include acrylic polymers (such as polymethyl methacrylate, polyethyl methacrylate, a methyl methacrylate-ethyl methacrylate copolymer, crosslinked polymethyl methacrylate, crosslinked polyethyl methacrylate, and polyamides), polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of the organic filler is not particularly limited, and the organic filler can be used by appropriately selecting the particle size of filler. In view of considerations such as the ease of handling and mechanical strength of the dental curable composition obtained, the organic filler (BP) has an average particle diameter of preferably 0.001 to 50 μm , more preferably 0.001 to 10 μm.

The content of the filler (B) in a dental curable composition of the present invention (the content of filler (B) after surface treatment when the filler (B) is surface treated) is not particularly limited. However, in view of considerations such as the ease of handling of the dental curable composition and the mechanical strength of the cued product obtained, the content of the filler (B) in a dental curable composition of the present invention is preferably 10 parts or more by mass, more preferably 30 parts or more by mass, even more preferably 50 parts or more by mass, particularly preferably 65 parts or more by mass, and is preferably 97 parts or less by mass, more preferably 96 parts or less by mass, even more preferably 95 parts or less by mass, particularly preferably 90 parts or less by mass in total 100 parts by mass of polymerizable monomer (A) and filler (B). With the content of filler (B) having the foregoing lower limits, it is possible to more effectively reduce stickiness or stringiness in the dental curable composition, and improve the ease of handling of the dental curable composition, in addition to improving the mechanical strength of the cured product. With the content of filler (B) having the foregoing upper limits, it is possible to prevent excessive hardening of the dental curable composition obtained, and the ease of handling improves.

In a dental curable composition of the present invention, the content of the inorganic fine particle (BF-1) (the content of inorganic fine particle (BF-1) after surface treatment when the inorganic fine particle (BF-1) is surface treated) is not particularly limited. However, in view of considerations such as the ease of handling of the dental curable composition obtained and the mechanical strength of the cued product, the content of the inorganic fine particle (BF-1) in a dental curable composition of the present invention is preferably 1 part or more by mass, more preferably 3 parts or more by mass, even more preferably 5 parts or more by mass, particularly preferably 10 parts or more by mass, and is preferably 95 parts or less by mass, more preferably 90 parts or less by mass, even more preferably 80 parts or less by mass, particularly preferably 70 parts or less by mass in total 100 parts by mass of polymerizable monomer (A) and filler (B). With the content of inorganic fine particle (BF-1) having the foregoing lower limits, it is possible to improve the ease of polishing of the cured product of the dental curable composition obtained, in addition to improving color compatibility and the mechanical strength of the cured product. With the content of inorganic fine particle (BF-1) having the foregoing upper limits, it is possible to prevent excessive hardening of the dental curable composition obtained, and the ease of handling improves.

Preferably, a dental curable composition of the present invention comprises the inorganic agglomerated particle (BF-2) and/or the organic-inorganic composite filler (BC). The total content of inorganic agglomerated particle (BF-2) and organic-inorganic composite filler (BC) (the content of inorganic agglomerated particle (BF-2) and organic-inorganic composite filler (BC) after surface treatment when these are surface treated) is not particularly limited. However, in view of considerations such as the ease of handling of the dental curable composition obtained and the mechanical strength of the cued product, the total content of the inorganic agglomerated particle (BF-2) and organic-inorganic composite filler (BC) in a dental curable composition of the present invention is preferably 1 part or more by mass, more preferably 2 parts or more by mass, even more preferably 3 parts or more by mass, particularly preferably 4 parts or more by mass, and is preferably 95 parts or less by mass, more preferably 90 parts or less by mass, even more preferably 80 parts or less by mass, particularly preferably 70 parts or less by mass in total 100 parts by mass of polymerizable monomer (A) and filler (B). With the total content of inorganic agglomerated particle (BF-2) and organic-inorganic composite filler (BC) having the foregoing lower limits, it is possible to more effectively reduce stickiness or stringiness, and the ease of handling improves. With the total content of inorganic agglomerated particle (BF-2) and organic-inorganic composite filler (BC) having the foregoing upper limits, it is possible to more effectively reduce roughness or runniness in the dental curable composition obtained, and the ease of handling improves.

Preferably, a dental curable composition of the present invention comprises the light diffusive inorganic agglomerated particle (BF-2d) or the light diffusive organic-inorganic composite filler (BC-d), or both. The total content of light diffusive inorganic agglomerated particle (BF-2d) and light diffusive organic-inorganic composite filler (BC-d) (the content of light diffusive inorganic agglomerated particle (BF-2d) and light diffusive organic-inorganic composite filler (BC-d) after surface treatment when these are surface treated) is not particularly limited. However, in view of considerations such as the ease of handling of the dental curable composition obtained and the mechanical strength of the cued product, and the aesthetics of the filled material, the total content of the light diffusive inorganic agglomerated particle (BF-2d) and light diffusive organic-inorganic composite filler (BC-d) in a dental curable composition of the present invention is preferably 0.5 parts or more by mass, more preferably 1 part or more by mass, even more preferably 2 parts or more by mass, particularly preferably 3 parts or more by mass, and is preferably 50 parts or less by mass, more preferably 45 parts or less by mass, even more preferably 40 parts or less by mass, particularly preferably 35 parts or less by mass in total 100 parts by mass of polymerizable monomer (A) and filler (B). With the total content of light diffusive inorganic agglomerated particle (BF-2d) and light diffusive organic-inorganic composite filler (BC-d) having the foregoing lower limits, it is possible to more effectively reduce stickiness or stringiness, and the ease of handling improves. It is also possible to impart sufficient light diffusion properties to the dental curable composition obtained, and provide a remarkably aesthetic restoration method, as noted above. With the total content of light diffusive inorganic agglomerated particle (BF-2d) and light diffusive organic-inorganic composite filler (BC-d) having the foregoing upper limits, it is possible to impart certain transparency, in addition to more effectively reducing roughness or runniness in the dental curable composition obtained. In this way, the dental curable composition, despite being a single composition, can provide a remarkably aesthetic restoration method for natural teeth over a wide range of shades, as noted above.

The content of the organic filler (BP) in a dental curable composition of the present invention is not particularly limited, and the organic filler (BP) may be present or absent. A dental curable composition containing the organic filler (BP) has reduced polymerization shrinkage upon cure. Because the mechanical strength and other properties of the cured product tend to decrease, a dental curable composition of the present invention comprises preferably 10 parts or less by mass of organic filler (BP), or may comprise 5 parts or less by mass, or 3 parts or less by mass of organic filler (BP), or no organic filler (BP), in total 100 parts by mass of polymerizable monomer (A) and inorganic filler (BF).

[Polymerization Initiator (C)]

A dental curable composition of the present invention comprises a polymerization initiator (C). The polymerization initiator (C) can be selected from polymerization initiators used in industry. Preferred for use are polymerization initiators used in dentistry. Particularly preferred are photopolymerization initiators and chemical polymerization initiators. The polymerization initiator (C) may be used alone, or two or more thereof may be used in an appropriate combination.

Examples of the photopolymerization initiators include (bis)acylphosphine oxides, ketals, $\alpha$-diketones, and coumarins.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, and salts of these (such as sodium salts, potassium salts, and ammonium salts). Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts of these (such as sodium salts, potassium salts, and ammonium salts).

Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the ketals include benzyl dimethyl ketal, and benzyl diethyl ketal.

Examples of $\alpha$-diketones include diacetyl, benzyl, camphorquinone, 2,3-pentadione, 2,3-octadione, 9,10-phenanthrenequinone, and 4,4'-oxybenzyl, acenaphthenequinone. Preferred is camphorquinone for its maximum absorption wavelength occurring in the visible light region.

Examples of the coumarin compounds include compounds mentioned in JP H09-3109 A and JP H10-245525 A, such as 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarin compounds are 3,3'-carbonylbis(7-diethylaminocoumarin), and 3,3'-carbonylbis(7-dibutylaminocoumarin).

By using at least one selected from the group of photopolymerization initiators consisting of an (bis)acylphosphine oxide, an α-diketone, and a coumarin compound, a dental curable composition can be obtained that has excellent photocurability both in the visible light region and the near ultraviolet region, and that shows sufficient photocurability regardless of whether the light source used is a halogen lamp, a light emitting diode (LED), or a xenon lamp. In view of obtaining a dental curable composition having even superior gloss retention, it is preferable to use a (bis)acylphosphine oxide and an α-diketone in combination. This is preferred because it improves the surface hardness of the cured product, though the mechanism remains unclear.

Preferred for use as chemical polymerization initiators are organic peroxides. The organic peroxides used as chemical polymerization initiators are not particularly limited, and may be known organic peroxides. Typical examples of organic peroxides include ketone peroxides, hydroperoxides, diacyl peroxides, dialkyl peroxides, peroxy ketals, peroxyesters, and peroxydicarbonates.

Examples of the ketone peroxides include methyl ethyl ketone peroxide, methyl isobutyl ketone peroxide, methyl cyclohexanone peroxide, and cyclohexanone peroxide.

Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxy ketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

Examples of the peroxyesters include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleic acid.

Examples of the peroxydicarbonates include di-3-methoxyperoxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

From an overall balance of safety, storage stability, and radical generating potential, preferred among these organic peroxides are diacyl peroxides, more preferably benzoyl peroxide.

The content of the polymerization initiator (C) in a dental curable composition of the present invention is not particularly limited. However, in view of considerations such as the curability of the dental curable composition obtained, the content of polymerization initiator (C) is preferably 0.001 parts or more by mass, more preferably 0.01 parts or more by mass, even more preferably 0.02 parts or more by mass, particularly preferably 0.1 parts or more by mass relative to total 100 parts by mass of polymerizable monomer (A). For considerations such as possible precipitation from the dental curable composition when the content of polymerization initiator (C) is too high, the content of polymerization initiator (C) is preferably 30 parts or less by mass, more preferably 20 parts or less by mass, even more preferably 15 parts or less by mass, particularly preferably 10 parts or less by mass, or may be 5 parts or less by mass, or 2 parts or less by mass, relative to total 100 parts by mass of polymerizable monomer (A).

[Colorant (D)]

A dental curable composition of the present invention comprises a colorant (D). The colorant (D) is not particularly limited, including the type of colorant (D). Any inorganic pigments and/or organic pigments may be used according to the intended shade of the dental curable composition. The shape of color particles is not particularly limited, and may be any of various shapes, including, for example, spherical, stylus, plate-like, crushed, and scale-like shapes. Specific examples of inorganic pigments include chromates such as chrome yellow, zinc yellow, and barium yellow; ferrocyanides such as iron blue; sulfides such as silver vermilion, cadmium yellow, zinc sulfide, and cadmium red; sulfates such as barium sulfate, zinc sulfate, and strontium sulfate; oxides such as zinc white, titanium white, antimony white, red iron oxide, iron black, and chromium oxide; hydroxides such as aluminum hydroxide; silicates such as calcium silicate, and ultramarine; and carbons such as carbon black, and graphite. Specific examples of organic pigments include nitroso pigments such as naphthol green B, and naphthol green Y; nitro pigments such as naphthol yellow S, and lithol fast yellow 2G; insoluble azo pigments such as permanent red 4R, brilliant fast scarlet, hansa yellow, and benzidine yellow; poorly soluble azo pigments such as lithol red, lake red C, and lake red D; soluble azo pigments such as brilliant carmine 6B, permanent red F5R, pigment scarlet 3B, and bordeaux 10B; phthalocyanine pigments such as phthalocyanine blue, phthalocyanine green, and sky blue; basic dye pigments such as rhodamine lake, malachite green lake, and methyl violet lake; and acidic dye pigments such as peacock blue lake, eosin lake, quinoline yellow lake, and aluminum lake. These colorants (D) may be used alone, or two or more thereof may be used in combination. Preferred among these colorants (D) are inorganic pigments such as titanium white, red iron oxide, iron black, and yellow ferrous oxide, which are superior to organic pigments in terms of properties such as heat resistance and lightfastness.

The content of the colorant (D) in a dental curable composition of the present invention is not particularly limited, as long as the present invention can exhibit its effects. However, in view of aesthetics, the content of colorant (D) is preferably 0.0005 parts or more by mass, more preferably 0.002 parts or more by mass, even more preferably 0.004 parts or more by mass, particularly preferably 0.006 parts or more by mass relative to 100 parts by mass of polymerizable monomer (A). With the content of colorant (D) having these lower limits, it is possible to effectively reduce a dark dull impression in filled portions. The content of colorant (D) is preferably 0.5 parts or less by mass, more preferably 0.4 parts or less by mass, even more preferably 0.3 parts or less by mass. With these upper limits, the shade of the cavity floor can effectively manifest in filled portions. The content of colorant (D) is preferably 0.00001 parts or more by mass, more preferably 0.0001 or more parts or more by mass, even more preferably 0.0005 parts or more by mass, particularly preferably 0.001 parts or more by mass relative to 100 parts by mass of the dental curable composition. The content of colorant (D) is preferably 0.3 parts or less by mass, more preferably 0.1 parts or less by mass, even more preferably 0.07 parts or less by mass.

[Polymerization Accelerator (E)]

A dental curable composition of the present invention may further comprise a polymerization accelerator (E). Examples of the polymerization accelerator include amines, sulfinic acids and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds. The polymerization accelerator (E) may be used alone, or two or more thereof may be used in combination.

The amines can be classified into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of curability and storage stability of the dental curable composition, preferred are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, 2-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart superior curability to the dental curable composition, preferred for use is at least one selected from the group consisting of N,N-bis(2-hydroxyethyl)-p-toluidine, ethyl 4-(N,N-dimethylamino)benzoate, 2-butoxyethyl 4-(N,N-dimethylamino)benzoate, and 4-(N,N-dimethylamino)benzophenone.

Examples of the sulfinic acids and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Preferred are sodium benzenesulfinate, sodium p-toluenesulfinate, and sodium 2,4,6-triisopropylbenzenesulfinate.

The borate compounds are preferably arylborate compounds. Examples of the arylborate compounds include borate compounds having one aryl group per molecule, borate compounds having two aryl groups per molecule, borate compounds having three aryl groups per molecule, and borate compounds having four aryl groups per molecule. In view of storage stability, preferred are borate compounds having three or four aryl groups per molecule.

Examples of the borate compounds having one aryl group per molecule include trialkylphenylboron, trialkyl(p-chlorophenyl)boron, trialkyl(p-fluorophenyl)boron, trialkyl[3,5-bis(trifluoromethyl)phenyl]boron, trialkyl[3,5-bis(1,1,1,3,3,3-hexafluoro-2-methoxy-2-propyl)phenyl]boron, trialkyl(p-nitrophenyl)boron, trialkyl(m-nitrophenyl)boron, trialkyl(p-butylphenyl)boron, trialkyl(m-butylphenyl)boron, trialkyl(p-butyloxyphenyl)boron, trialkyl(m-butyloxyphenyl)boron, trialkyl(p-octyloxyphenyl)boron, trialkyl(m-octyloxyphenyl)boron (the alkyl group is at least one selected from the group consisting of, for example, n-butyl, n-octyl, and n-dodecyl), and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having two aryl groups per molecule include dialkyldiphenylboron, dialkyl di(p-chlorophenyl)boron, dialkyl di(p-fluorophenyl)boron, dialkyl di[3,5-bis(trifluoromethyl)phenyl]boron, dialkyl di[3,5-bis(1,1,1,3,3,3-hexafluoro-2-methoxy-2-propyl)phenyl]boron, dialkyl di(p-nitrophenyl)boron, dialkyl di(m-nitrophenyl)boron, dialkyl di(p-butylphenyl)boron, dialkyl di(m-butylphenyl)boron, dialkyl di(p-butyloxyphenyl)boron, dialkyl di(m-butyloxyphenyl)boron, dialkyl di(p-octyloxyphenyl)boron, dialkyl di(m-octyloxyphenyl)boron (the alkyl groups in these examples are, for example, n-butyl, n-octyl, or n-dodecyl), and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having three aryl groups per molecule include monoalkyl triphenylboron, monoalkyl tri(p-chlorophenyl)boron, monoalkyl tri(p-fluorophenyl)boron, monoalkyl tri[3,5-bis(trifluoromethyl)phenyl]boron, monoalkyl tri[3,5-bis(1,1,1,3,3,3-hexafluoro-2-methoxy-2-propyl)phenyl]boron, monoalkyl tri(p-nitrophenyl)boron, monoalkyl tri(m-nitrophenyl)boron, monoalkyl tri(p-butylphenyl)boron, monoalkyl tri(m-butylphenyl)boron, monoalkyl tri(p-butyloxyphenyl)boron, monoalkyl tri(m-butyloxyphenyl)boron, monoalkyl tri(p-octyloxyphenyl)boron, monoalkyl tri(m-octyloxyphenyl)boron (the alkyl group is one selected from, for example, n-butyl, n-octyl, and n-dodecyl), and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having four aryl groups per molecule include tetraphenyl boron, tetrakis(p-chlorophenyl)boron, tetrakis(p-fluorophenyl)boron, tetrakis[3,5-bis(trifluoromethyl)phenyl]boron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, [3,5-bis(trifluoromethyl)phenyl]triphenylboron, (p-nitrophenyl)triphenylboron, (m-butyloxyphenyl)triphenylboron, (p-butyloxyphenyl)triphenylboron, (m-octyloxyphenyl)triphenylboron, (p-octyloxyphenyl)triphenylboron, and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the derivatives of barbituric acid include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, thiobarbituric acid, and salts of these (particularly preferred are alkali metal salts or alkali earth metal salts). Particularly preferred as derivatives of barbituric acid are, for example, 5-butylbarbituric acid, 1,3,5-trimethylbarbituric acid, 1-cyclohexyl-5-ethylbarbituric acid, 1-benzyl-5-phenylbarbituric acid, and sodium salts of these.

Examples of the triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-($\alpha,\alpha,\beta$-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N, N-bis(2-hydroxyethyl)am ino}ethoxy]-4,6-bis(trichloromethyl)-s-triazine, 2-[2-{N-hydroxyethyl-N-ethylam ino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

In view of polymerization activity, preferred among these triazine compounds is 2,4,6-tris(trichloromethyl)-s-triazine. In view of storage stability, preferred are 2-phenyl-4,6-bis(trichloromethyl)-s-triazine, 2-(p-chlorophenyl)-4,6-bis(trichloromethyl)-s-triazine, and 2-(4-biphenylyl)-4,6-bis(trichloromethyl)-s-triazine. The triazine compounds may be used alone, or two or more thereof may be used in combination.

Preferred for use as copper compounds are, for example, copper acetylacetonate, copper(II) acetate, copper oleate, copper(II) chloride, and copper(II) bromide.

Examples of the tin compounds include di-n-butyltin dimaleate, di-n-octyltin dimaleate, di-n-octyltin dilaurate, and di-n-butyltin dilaurate. Particularly preferred as tin compounds are di-n-octyltin dilaurate and di-n-butyltin dilaurate.

The vanadium compounds are preferably vanadium compounds with valences of IV and/or V. Examples of vanadium compounds with valences of IV and/or V include compounds mentioned in JP 2003-96122 A, for example, such as vanadium(IV) oxide, vanadium(IV)oxy acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate.

Preferred for use as halogen compounds are, for example, dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the aldehydes include terephthalaldehyde, and derivatives of benzaldehyde. Examples of derivatives of benzaldehyde include dimethylaminobenzaldehyde, p-methoxybenzaldehyde, p-ethoxybenzaldehyde, and p-n-octyloxybenzaldehyde. In view of curability, preferred for use is p-n-octyloxybenzaldehyde.

Examples of the thiol compounds include 3-mercaptopropyltrimethoxysilane, 2-mercaptobenzoxazole, decanethiol, and thiobenzoic acid.

Examples of the sulfites include sodium sulfite, potassium sulfite, calcium sulfite, and ammonium sulfite.

Examples of the bisulfites include sodium bisulfite and potassium bisulfite.

Examples of the thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, and tetracyclohexylthiourea.

When a dental curable composition of the present invention comprises the polymerization accelerator (E), the content of polymerization accelerator (E) is not particularly limited. However, in view of considerations such as curability of the dental curable composition obtained, the content of polymerization accelerator (E) is preferably 0.001 parts or more by mass, more preferably 0.01 parts or more by mass, even more preferably 0.02 parts or more by mass, or may be 0.03 parts or more by mass, 0.05 parts or more by mass, or 0.1 parts or more by mass, relative to total 100 parts by mass of polymerizable monomer (A). For considerations such as possible precipitation from the dental curable composition when the content of polymerization accelerator (E) is too high, the content of polymerization accelerator (E) is preferably 30 parts or less by mass, more preferably 20 parts or less by mass, even more preferably 10 parts or less by mass, particularly preferably 5 parts or less by mass, or may be 2 parts or less by mass, 1 part or less by mass, or 0.5 parts or less by mass, relative to total 100 parts by mass of polymerizable monomer (A).

[Additive (F)]

A dental curable composition of the present invention may optionally comprise an additive (F), for example, such as a polymerization inhibitor, a chain transfer agent, a ultraviolet absorber, an antioxidant, an antimicrobial agent, a dispersant, or a pH adjuster, other than the polymerizable monomer (A), filler (B), polymerization initiator (C), colorant (D), and polymerization accelerator (E) described above. The additive (F) may be used alone, or two or more thereof may be used in combination.

Examples of the polymerization inhibitor include 3,5-di-t-butyl-4-hydroxytoluene, hydroquinone, dibutyl hydroquinone, dibutyl hydroquinone monomethyl ether, hydroquinone monomethyl ether, and 2,6-di-t-butylphenol. These may be used alone, or two or more thereof may be used in combination. Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(2-hydroxy-3-tert-butyl-5-methylphenyl)-5-chloro-2H-benzotriazole (Tinuvin 326); and benzoimidazole compounds. These may be used alone, or two or more thereof may be used in combination.

As described above, a dental curable composition of the present invention, despite being a single dental curable composition, shows good color compatibility with natural teeth over a wide range of shades in cases involving cavity floors in different classes of cavities, such as Class I, Class II, and Class V, and a cured product of a dental curable composition of the present invention excels in ease of polishing and gloss retention. Within the range of the descriptions of the present specification, an embodiment may be selected that focuses on gloss retention in particular. For example, in terms of focusing on gloss retention in particular, a certain embodiment (Y-1) may be, for example, a dental curable composition in which the color compatibility ($\Delta E^*$ represented by the formula below) with Class I cavities is 6.0 or less for both shade A1 and shade A4 of a shade guide (The VITA Classical Shade Guide manufactured by VITA Zahnfabrik, Germany, under this trade name), and that has a gloss retention of 40% or more.

$$\Delta E^* = ((L^*_1 - L^*_0)^2 + (a^*_1 - a^*_0)^2 + (b^*_1 - b^*_0)^2)^{1/2}$$

The symbols in the formula are as described in the EXAMPLES section below.

The method of measurement of color compatibility with Class I cavities, and the method of measurement of gloss retention in embodiment (Y-1) are as described in the EXAMPLES section below.

As noted above, a dental curable composition of the present invention, despite being a single dental curable composition and without changing its composition, shows good color compatibility with natural teeth over a wide range of shades. Accordingly, in certain embodiments, a dental curable composition of the present invention has excellent color compatibility ($\Delta E^*$) with both shade A1 and shade A4 of the shade guide in cases involving Class I cavities.

Changes may be appropriately made to the dental curable composition of embodiment (Y-1) based on the descriptions of the present specification.

The composition in the dental curable composition of embodiment (Y-1) is not particularly limited, as long as the color compatibility with Class I cavities, and the gloss retention are confined within predetermined ranges. For example, the dental curable composition of embodiment (Y-1) preferably comprises a polymerizable monomer (A). The dental curable composition of embodiment (Y-1) preferably comprises a filler (B). The dental curable composition of embodiment (Y-1) preferably comprises a polymerization initiator (C). The dental curable composition of embodiment (Y-1) preferably comprises a colorant (D). The dental curable composition of embodiment (Y-1) may or may not comprise a colorant (D). For example, a dental curable composition of the present invention also encompasses a dental curable composition that does not comprise a colorant (D), and that, despite being a single dental curable composition and without changing its composition, shows good color compatibility with natural teeth over a wide range of shades, and excels in gloss retention with the spectral reflectance ratios $R_{650/600}$, $R_{700/600}$, and $R_{750/600}$ all confined within the predetermined ranges by appropriately combining different types of polymerization initiators (C) (for example, use of (bis)acylphosphine oxides and $\alpha$-diketones in combination as photopolymerization initiators), different types of fillers (B), different sizes (e.g., average particle diameter) and different shapes (for example, irregularly shaped fillers), and different production methods (for example, a surface treatment with a surface treatment agent, and a heat treatment).

In embodiment (Y-1), the gloss retention is preferably 55% or more, more preferably 60% or more, even more preferably 65% or more.

In embodiment (Y-1), the color compatibility ($\Delta E^*$) with Class I cavities is preferably 5.5 or less, more preferably 5.0 or less, even more preferably 4.5 or less for both shade A1 and shade A4 of a shade guide (The VITA Classical Shade Guide manufactured by VITA Zahnfabrik, Germany, under this trade name).

<<Method of Production of Dental Curable Composition>>

The method of preparation of a dental curable composition of the present invention is not particularly limited, and a dental curable composition of the present invention can be obtained by adding each component in a predetermined amount. The order in which components are added is not particularly limited, and the components may be added at once, or may be added in two or more separate portions. Optionally, the components may be mixed or kneaded, or may be subjected to defoaming, for example, vacuum defoaming. The resultant dental curable composition may be filled into a single container (e.g., a syringe) to prepare a one-pack (one-paste) type dental curable composition.

«Uses»

A dental curable composition of the present invention is not limited to particular uses, and may be used for a variety of dental materials. Specifically, a dental curable composition of the present invention can be suitably used as, for example, a dental composite resin (for example, a composite resin for filling cavities, a composite resin for abutment construction, a composite resin for dental caps, a self-adhesive composite resin), a denture base resin, a denture base liner, an impression material, a luting material (for example, a resin cement, a resin-added glass ionomer cement), a dental bonding agent (for example, an orthodontic adhesive, an adhesive for application to cavities), a tooth fissure sealant, a resin block for CAD/CAM, a temporary crown, or an artificial teeth material. Because of high aesthetics and superior mechanical strength, a dental curable composition of the present invention is particularly suited for dental composite resins, and resin blocks for CAD/CAM.

The present invention encompasses embodiments combining the foregoing features, provided that the present invention can exhibit its effects with such combinations made in various forms within the technical idea of the present invention.

EXAMPLES

The following describes the present invention in greater detail by way of Examples and Comparative Examples. It is to be noted, however, that the present invention is not limited to the following EXAMPLES. Details are summarized below, including the test methods and materials used in EXAMPLES.

«Test Methods»

[Average Particle Diameter of Filler]

The fillers below were measured for average particle diameter by volume with a laser diffraction particle size distribution analyzer (SALD-2300 manufactured by Shimadzu Corporation), using ethanol as dispersion medium (specifically, for the measurement of particles of 0.1 μm or more). Alternatively, a scanning electron microscope (SU3500 manufactured by Hitachi High-Technologies Corporation) was also used for the measurement of average particle diameter (n = 1).

[Refractive Index of Filler]

For the fillers below, a dispersion was prepared by dispersing a certain amount (0.1 g/mL) of each filler in an organic solvent of a known refractive index (a mixture of two or more organic solvents selected from the group consisting of 1-bromonaphthalene, methyl salicylate, dimethylformamide, and 1-pentanol; adjusted to various refractive indices). The refractive index (nD25) of organic solvent at which the dispersion shows the maximum transmittance for 589 nm wavelength of light was then determined as the refractive index of the filler. An organic solvent of a known refractive index can be selected according to the type of filler being measured for refractive index, based on an expected refractive index. The refractive index of organic solvent was measured with an abbe refractometer (NAR-1T LIQUID manufactured by Atago Co., Ltd. under this trade name), using a Na-D illuminant. The transmittance of the dispersion was measured in a quartz cell with a 10 mm light path, using a ultraviolet-visible spectrophotometer (UV-2400 manufactured by Shimadzu Corporation under this trade name).

[Refractive Index of Polymer]

The refractive index of a polymer of a polymerizable monomer was measured in a 25°C thermostatic chamber, using the same abbe refractometer used above. Specifically, a uniform mixture containing a polymerizable monomer was placed in a die having a hole measuring 10 mm in diameter × 1.0 mm after the mixture was prepared for Examples and Comparative Examples of Table 1 by mixing the polymerizable monomer (A), polymerization initiator (C), polymerization accelerator (E), and additive (F) in the proportions shown in Table 1. After placing glass slides on both sides with pressure, the mixture was cured by applying light from both sides, 45 seconds on each side, with an LED photopolymerizer (α Light V manufactured by J. Morita Corp.; wavelength: 400 to 408 nm, 465 to 475 nm). After cure, the cured product of the polymerizable monomer-containing mixture was taken out of the die. In setting the cured product on the abbe refractometer, a solvent (1-bromonaphthalene) that does not dissolve the specimen and having a higher refractive index than the specimen was dropped on the specimen to more closely contact the cured product with the measurement surface.

[Specific Surface Area of Inorganic Filler]

The inorganic filler obtained in each Production Example below was degassed in a vacuum at 100°C for 2 hours, and the specific surface area of the inorganic filler was measured by the BET method with a specific surface area measurement device (BELSORP-mini II manufactured by MicrotracBEL Corp.), using nitrogen as adsorbate gas and at a measurement temperature of 77 K (n = 1). For the measurement, a multi-point BET analysis was adopted by taking 5 points on the adsorption isotherm in the pressure ($P/P_0$) range of 0.05 to 0.3, where P is the adsorbate equilibrium pressure (kPa), and $P_0$ is the saturated vapor pressure (kPa)

[Spectral Reflectance Ratios ($R_{650/600}$, $R_{700/600}$, $R_{750/600}$)]

The spectral reflectance ratios of the cured product were evaluated with a spectral colorimeter (SE6000 manufactured by Nippon Denshoku Industries Co., Ltd., illuminant: D65/2, measurement window: $\varnothing$ = 6 mm). Specifically, the dental curable composition was filled into a stainless-steel die (10 mm in diameter × 1 mm in thickness). With glass slides placed on top and bottom with pressure, the dental curable composition was cured by applying light to both sides, 45 seconds on each side, with an LED photopolymerizer ($\alpha$ Light V manufactured by J. Morita Corp.; wavelength: 400 to 408 nm, 465 to 475 nm). After being taken out of the die, the cured plate was measured for spectral reflectance against 380 to 780 nm wavelengths with a standard whiteboard (X = 93.94, Y = 95.90, Z = 112.92) configured and placed behind the cured plate (n = 1). The spectral reflectance ratios ($R_{650/600}$, $R_{700/600}$, $R_{750/600}$) were then calculated from the formulae described above.

[Contrast Ratio]

The contrast ratio of the cured product was evaluated with the spectral colorimeter above. Specifically, the same method was used to prepare a cured plate measuring 10 mm in diameter and 1 mm in thickness, and the Y value of tristimulus values was measured against a black background and a white background. A standard whiteboard was used as the white background, and a matte dark box as the black background. The contrast ratio was calculated from the formula described above (n = 1).

[Light Diffusivity LD]

The light diffusivity LD of the cured product was evaluated with a goniophotometer (GP-200 manufactured by Murakami Color Research Laboratory Co., Ltd.). Specifically, the dental curable composition was placed and pressed between cover glasses from top and bottom with 0.25 mm-thick stainless-steel spacers, and was cured into a cured plate (30 mm in diameter, 0.25 mm thick) by applying light from both sides, 45 seconds on each side, with an LED photopolymerizer ($\alpha$ Light V manufactured by J. Morita Corp.; wavelength: 400 to 408 nm, 465 to 475 nm). The cured plate was then measured for the luminous intensity distribution of transmitted light from -90° to +90° relative to an incident light angle of 0°, using the goniophotometer. The light diffusivity LD was calculated following the formula (2) described above (n = 1).

[Lightness (L*/w) and Chromaticity Indices (a*/w, b*/w)]

The lightness and chromaticity of the cured product were evaluated using the same spectral colorimeter (SE6000 manufactured by Nippon Denshoku Industries Co., Ltd., illuminant: D65/2, measurement window: $\varnothing$ = 6 mm) used for the measurement of spectral reflectance ratio. Specifically, the same method was used to prepare a cured plate measuring 10 mm in diameter and 1 mm in thickness, and the lightness (L*/w) and chromaticity indices (a*/w, b*/w) were measured in L*a*b*color system with a standard whiteboard placed behind the cured plate (n = 1).

[Evaluation of Ease of Handling]

The composition was filled into a plastic simulated cavity (a hole simulating a Class 5 cavity, 3 mm in diameter and 2 mm in depth) at 25°C using a metallic filling instrument, and the ease of filling operation was evaluated. Specifically, for dental curable compositions that were clay-like in characteristics, the dental curable composition of each Example and Comparative Example was filled into the simulated cavity with a metallic dental plugger (condensers #4 and #5 manufactured by Yoshida Dental Trade Distribution Co., Ltd.), and the stickiness feel of the dental curable composition was evaluated following the criteria below. For dental curable compositions that were liquid-like in characteristics, the dental curable composition was filled into the barrel of a syringe (the barrel of the CLEARFIL MAJESTY ES Flow syringe manufactured by Kuraray Noritake Dental Inc.), and the dental curable composition was pushed into the simulated cavity from the barrel with the accessory needle tip attached to the syringe. The stringiness of the dental curable composition was then evaluated according to the following criteria.

Good: No stickiness feel or stringiness
Moderate: Slight stickiness feel or stringiness
Poor: Serious stickiness feel or stringiness

[Color Compatibility with Class I Cavity]

The dental curable composition was evaluated for its molar color compatibility by filling and restoring a cavity formed in an artificial tooth, and measuring the restored portion with a dental colorimeter. Specifically, first, an artificial molar #6 (Zen Opal molar, size: PL16, shade: A1, A3, and A4; manufactured by GC JAPAN) was photographed with a dental colorimeter (Crystaleye Spectrophotometer manufactured by Olympus Corporation). Here, the artificial tooth was measured in the dark box (check box, top cover) attached to the dental colorimeter. Thereafter, a Class I cavity, measuring 4 mm in diameter and 2 mm in depth, was formed in the center of the artificial tooth, and the cavity surface was etched with a dental etchant (K etchant GEL manufactured by Kuraray Noritake Dental Inc.), according to the method recommended by the manufacturer (the method described in the package insert). This was followed by bonding of the cavity surface with a dental adhesive (Clearfil Universal Bond Quick ER manufactured by Kuraray Noritake Dental Inc.) according to the method recommended by the manufacturer, and irradiation with a dental visible light irradiator (PenCure2000 manufactured by J. Morita Corp.) in normal mode for 10 seconds. The cavity was then filled with the dental curable composition, and an artificial tooth mold, prepared in advance with a silicone impression material (Memosil 2 manufactured by Heraeus Kulzer Japan), was pressed against the dental curable composition to give the shape of the artificial tooth. The filled portion was polished for 30 seconds with a dental rubber polisher (CompoMaster, CA, 13S manufactured by Shofu Inc.) under supplied water at a rotational speed of about 10,000 rpm, using a dental unit (PORTACARE 21 manufactured by J. Morita Corp.). Finally, the dental curable composition was irradiated with light for 10 seconds over the mold, using the dental visible light irradiator in normal mode. After removing the mold, the dental curable composition was cured into a filling specimen by applying light for 10 seconds in normal mode. The specimen was photographed using the same method used to take an image of the artificial molar. The filled portion was then measured for its lightness ($L^*_1$) and chromaticity ($a^*_1$, $b^*_1$) in the captured image of the filled specimen, using the software that comes with the dental colorimeter. The image of untreated artificial tooth captured beforehand was also measured for lightness ($L^*_0$) and chromaticity ($a^*_0$, $b^*_0$) in the same areas measured for the image of the artificial tooth filling specimen. The color difference $\Delta E^*$ was then calculated as an index of color compatibility, using the following formula (n = 1).

$$\Delta E^* = ((L^*_1 - L^*_0)^2 + (a^*_1 - a^*_0)^2 + (b^*_1 - b^*_0)^2)^{1/2}$$

The preferred value of $\Delta E^*$ is 6.0 or less, more preferably 5.5 or less, even more preferably 5.0 or less for all shade A1, shade A3, and shade A4 of the artificial tooth.

[Color Compatibility to Class IV Cavity]

The dental curable composition was evaluated for its front-tooth color compatibility by filling and restoring a cavity formed in a shade guide, and measuring the restored portion with a dental colorimeter. Specifically, first, a shade guide (The VITA Classical Shade Guide, shade: A1, A3, and A4, manufactured by VITA Zahnfabrik, Germany) was photographed with the dental colorimeter, using the same method described above. Thereafter, a Class IV cavity, quadrantal in shape ($\varnothing$ = 4 mm) and penetrating from the tongue side to labial side, was formed at the incisal edge of the shade guide. The filling specimen was prepared using the same method described above. Similarly, the color difference $\Delta E^*$ was calculated as an index of color compatibility in the same fashion (n = 1). The preferred value of $\Delta E^*$ for the shade guide is 7.0 or less for A1, more preferably 6.0 or less for A3, even more preferably 5.0 or less for A4. Particularly preferably, $\Delta E^*$ is 6.0 or less for all A1, A3, and A4 of the shade guide.

[Ease of Polishing of Cured Product]

The dental curable composition was filled into a polytetrafluoroethylene mold (10 mm in diameter $\times$ 2.0 mm in thickness), and irradiated with light for 10 seconds with a dental visible light irradiator (PenCure2000 manufactured by J. Morita Corp.). The cured product was then taken out of the mold as a specimen. A flat surface of the specimen was ground with #600 abrasive paper under dry conditions, and was polished for 10 seconds with a dental rubber polisher (CompoMaster, CA, 13S manufactured by Shofu Inc.) under supplied water at a rotational speed of about 10,000 rpm, using a dental unit (PORTACARE 21 manufactured by J. Morita Corp.). The glossiness of the polished surface was then measured with a glossmeter (VG2000 manufactured by Nippon Denshoku Industries Co., Ltd.; measured at 60 degree angle in compliance with JIS Z 8741: 1997), and a fraction (glossiness) relative to the glossiness of a mirror at 100% was determined as an index of the ease of polishing of the cured product (n = 3). The mean values of measured values are presented in Table 2. The preferred glossiness is 20% or more, more preferably 25% or more, even more preferably 30% or more.

[Gloss Retention of Cured Product]

Gloss retention was evaluated based on glossiness changes before and after an abrasiveness test, specifically as follow. The dental curable composition (paste) was filled into a SUS mold (30 mm in length, 20 mm in width, 2 mm in thickness), and glass slides were placed on the top and bottom of the paste with pressure (over 30 mm × 20 mm surfaces). The paste was then cured by applying light from the top and bottom of the paste, 180 seconds on each side, over the glass slides, using a dental laboratory visible light irradiator (α Light V manufactured by J. Morita Corp.). By using the resulting cured product as a specimen, a specimen surface was polished with #1500 wet abrasive paper to provide a polished surface. The polished surface was buffed with a laboratory polishing box (EWL80 manufactured by KaVo) at 3,000 rpm for 20 seconds. The specimen was subjected to an abrasiveness test after buffing. For buffing, a dental polisher (PORCENY HYDON manufactured by TOKYO SHIZAISHA under this trade name) was used. The glossiness (G1) of the specimen before abrasiveness test was determined as a fraction relative to the glossiness of a mirror at 100%, using a glossmeter (VG2000 manufactured by Nippon Denshoku Industries Co., Ltd.; measured in compliance with JIS Z 8741:1997). Measurements were made at 60 degree angle. The specimen was subjected to 40,000 cycles of abrasiveness test under a load of 250 g, using a toothbrush abrasion tester (manufactured by DAIEI KAGAKU SEIKI MFG Co., Ltd.) with a suspension of toothpaste and a commercially available toothbrush (Between manufactured by Lion Corporation; size: regular; hardness: normal). The suspension of toothpaste was prepared from a commercially available toothpaste (Dentor Clear Max manufactured by Lion Corporation) in a toothpaste-to-distilled water ratio of 10/90 by mass. After the abrasiveness test, the specimen surface was measured for glossiness (G2), using the same method used for the measurement of glossiness before abrasiveness test. The gloss retention (%) was calculated as {(G2) × 100}/(G1) from the surface glossiness values of the specimen before and after the abrasiveness test. The preferred gloss retention is 40% or more, more preferably 45% or more, even more preferably 50% or more, particularly preferably 55% or more, 60% or more, or 65% or more.

«Materials»

(Polymerizable Monomer (A))

Bis-GMA: 2,2-Bis[4-(3-methacryloyloxy-2-hydroxypropoxy)phenyl]propane
D2.6E: 2,2-Bis(4-methacryloyloxypolyethoxyphenyl)propane (average number of moles of ethyleneoxy group added: 2.6)
UDMA: 2,2,4-Trimethylhexamethylene bis(2-carbamoyloxyethyl)dimethacrylate
DD: 1,10-Decanediol dimethacrylate
3G: Triethylene glycol dimethacrylate
POBMA: m-Phenoxybenzyl methacrylate

(Filler (B))

The fillers obtained in the Production Examples below were used. Commercially available fillers were also used, without any pre-conditioning.

[Production Example 1]

• Production of Inorganic Fine Particle (BF-1-1)

A three-neck flask was charged with 100 parts by mass of a commercially available barium glass (8235 UF0.7 manufactured by Schott; average particle diameter 0.7 $\mu$m , refractive index: 1.55), 2 parts by mass of 3-methacryloyloxypropyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain an inorganic fine particle (BF-1-1) having a surface treatment layer. The inorganic fine particle (BF-1-1) produced had an average particle diameter of 0.7 $\mu$m.

[Production Example 2]

• Production of Inorganic Fine Particle (BF-1-2)

A three-neck flask was charged with 100 parts by mass of a commercially available barium glass (GM27884 NanoFine180 manufactured by Schott; average particle diameter 0.2 $\mu$m , refractive index: 1.53), 11 parts by mass of 3-methacryloyloxypropyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain an inorganic fine particle (BF-1-2) having a surface treatment layer. The inorganic fine particle (BF-1-2) produced had an average particle diameter of 0.2 $\mu$m.

[Production Example 3]

• Production of Inorganic Fine Particle (BF-1-3)

A three-neck flask was charged with 100 parts by mass of a commercially available barium glass (GM27884 NanoFine180 manufactured by Schott; average particle diameter 0.2 $\mu$m , refractive index: 1.53), 11 parts by mass of 8-methacryloyloxyoctyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain an inorganic fine particle (BF-1-3) having a surface treatment layer. The inorganic fine particle (BF-1-3) produced had an average particle diameter of 0.2 $\mu$m.

[Production Example 4]

• Production of Inorganic Fine Particle (BF-1-4)

A three-neck flask was charged with 100 parts by mass of a commercially available barium glass (GM27884 NanoFine180 manufactured by Schott; average particle diameter 0.2 $\mu$m , refractive index: 1.53), 11 parts by mass of 11-methacryloyloxyundecyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain an inorganic fine particle (BF-1-4) having a surface treatment layer. The inorganic fine particle (BF-1-4) produced had an average particle diameter of 0.2 $\mu$m.

[Production Example 5]

· Production of Inorganic Fine Particle (BF-1-5)

A commercially available surface-treated silica-coated ytterbium fluoride (SG-YBF100WSCMP10 manufactured by Sukgyung AT; average particle diameter of primary particles: 110 nm, refractive index: 1.54) was used without any pre-conditioning.

[Production Example 6]

• Production of Additional Inorganic Agglomerated Particle (BF-2e-1)

Water in a commercially available silica-ytterbium oxide aqueous dispersion (SG-YBSO30SW manufactured by Sukgyung AT; average particle diameter of primary particles: 30 nm) was removed by distillation with an evaporator, and the resultant solid component was pulverized with a planetary ball mill (Classic Line P-6, zirconia ball manufactured by Fritsch, Germany) for 180 minutes. The powder obtained was fired for 1 hour with an electric furnace that had been set to 800°C, and pulverized with the planetary ball mill for 180 minutes. The resultant powder was hydrophobized by a surface treatment performed with 10 parts by mass of 3-methacryloyloxypropyltrimethoxysilane with respect to 100 parts by mass of the powder. This produced an additional inorganic agglomerated particle (BF-2e-1). The inorganic agglomerated particle (BF-2e-1) produced had an average particle diameter of 5.7 $\mu$m , a refractive index of 1.53, and a specific surface area of 95.8 m$^2$/g.

[Production Example 7]

• Production of Light Diffusive Inorganic Agglomerated Particle (BF-2d-1)

A three-neck flask was charged with 100 parts by mass of aggregated silica (Silica Micro Bead P-500 manufactured by JGC C & C; average particle diameter of primary particles: 12 nm, average particle diameter of aggregates: 2 $\mu$m), 20 parts by mass of 3-methacryloxypropyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain a sliane-treated light diffusive inorganic agglomerated particle (BF-2d-1). The silane-treated light diffusive inorganic agglomerated particle (BF-2d-1) had an average particle diameter of 1.6 $\mu$m , a refractive index of 1.44, a specific surface area of 99 m$^2$/g, and a pore volume of 0.19 mL/g.

[Production Example 8]

• Production of Light Diffusive Organic-Inorganic Composite Filler (BC-d-1)

A polymerizable monomer-containing composition was obtained by evenly dissolving 70 parts by mass of Bis-GMA, 30 parts by mass of 3G, and 0.5 parts by mass of benzoyl peroxide. Separately, a colloidal silica powder (Aerosil® OX50 manufactured by Nippon Aerosil Co., Ltd.; average particle diameter 0.04 $\mu$m) was surface treated with $\gamma$-methacryloxypropyltrimethoxysilane by an ordinary method. A paste-like composition was obtained by kneading 100 parts by mass of the surface-treated colloidal silica with 100 parts by mass of the polymerizable monomer. The composition was heated to polymerize at 130°C for 3 hours under reduced pressure, and the resultant cured product was pulverized with a ball mill to obtain a surface-untreated organic-inorganic composite filler. The surface-untreated organic-inorganic composite filler was surface treated with 1 part by mass of $\gamma$-methacryloxypropyltrimethoxysilane with respect to 100 parts by mass of the surface-untreated organic-inorganic composite filler. This produced a light diffusive organic-inorganic composite filler (BC-d-1). The light diffusive organic-inorganic composite filler (BC-d-1) had an average particle diameter of 11 $\mu$m , and a refractive index of 1.50.

[Production Example 9]

• Production of Light Diffusive Organic-Inorganic Composite Filler (BC-d-2)

A polymerizable monomer-containing composition was obtained by evenly dissolving 70 parts by mass of UDMA, 30 parts by mass of DD, and 0.5 parts by mass of benzoyl peroxide. Separately, a colloidal silica powder (Aerosil® OX50 manufactured by Nippon Aerosil Co., Ltd.; average particle diameter 0.04 $\mu$m) was surface treated with $\gamma$-methacryloxypropyltrimethoxysilane by an ordinary method. A paste-like composition was obtained by kneading 100 parts by mass of the surface-treated colloidal silica with 100 parts by mass of the polymerizable monomer. The composition was heated to polymerize at 130°C for 3 hours under reduced pressure, and the resultant cured product was pulverized with a ball mill to obtain a surface-untreated organic-inorganic composite filler. The surface-untreated organic-inorganic composite filler was surface treated with 1 part by mass of $\gamma$-methacryloxypropyltrimethoxysilane with respect to 100 parts by mass of the surface-untreated organic-inorganic composite filler. This produced a light diffusive organic-inorganic composite filler (BC-d-2). The light diffusive organic-inorganic composite filler (BC-d-2) had an average particle diameter of 15 $\mu$m , and a refractive index of 1.49.

[Production Example 10]

• Production of Organic-Inorganic Composite Filler (BC-e-1)

In order to produce a paste, 100 parts by mass of the inorganic fine particle (BF-1-2) produced above was added and mixed as inorganic filler into 100 parts by mass of a polymerizable monomer mixture of Bis-GMA and 3G (mass ratio 1:1) dissolving 1 mass% of azobisisobutyronitrile (AIBN) as polymerization initiator. The paste was subjected to thermal polymerization at 100°C for 5 hours in a reduced pressure atmosphere. The resultant cured product of polymerization was pulverized with a vibration ball mill to an average particle diameter of about 5 $\mu$m. The resultant powder (100 g) was surface treated by being refluxed at 90°C for 5 hours in a 200 mL ethanol solution containing 2 mass% of $\gamma$-methacryloyloxypropyltrimethoxysilane. This produced an organic-inorganic composite filler (BC-e-1). The organic-inorganic composite filler (BC-e-1) had an average particle diameter of 5.2 $\mu$m , and a refractive index of 1.54.

[Production Example 11]

• Production of Additional Inorganic Particle (BF-3-1)

A three-neck flask was charged with 100 parts by mass of a commercially available barium glass (8235 UF1.5 manufactured by Schott; average particle diameter 1.5 $\mu$m , refractive index: 1.55), 2 parts by mass of 3-methacryloyloxypropyltrimethoxysilane, and 170 parts by mass of toluene, and the mixture was stirred at room temperature for 2 hours. After removing toluene by distillation under reduced pressure, the mixture was dried at 40°C for 16 hours in a vacuum, and heated at 90°C for 3 hours to obtain an inorganic particle (BF-3-1) having a surface treatment layer. The inorganic particle (BF-3-1) produced had an average particle diameter of 1.5 $\mu$m.

(Polymerization Initiator (C))

CQ: Camphorquinone
TPO: 2,4,6-Trimethylbenzoyldiphenylphosphine oxide

(Colorant (D))

D-1: Titanium oxide
D-2: Iron black
D-3: Yellow ferrous oxide
D-4: Red iron oxide

(Polymerization accelerator (E))

PDE: Ethyl 4-(N,N-dimethylamino)benzoate

(Additive (F))

TIN: Tinuvin 326 (manufactured by BASF Japan; ultraviolet absorber)
BHT: 3,5-Di-t-butyl-4-hydroxytoluene (polymerization inhibitor)

[Examples 1 to 10 and Comparative Examples 1 to 5] (Dental Composite Resin) The materials shown in Table 1 were mixed and kneaded at an ordinary temperature (23°C) in the dark in the proportions shown in the table. After homogenization, the mixture was defoamed in a vacuum to prepare dental curable compositions. The dental curable compositions were tested using the methods described above. The results are presented in Table 2.

[Table 1]

| Components (parts by mass) | | | Ex. 1 | Ex. 2 | Ex. 3 | Ex. 4 | Ex. 5 | Ex. 6 | Ex. 7 | Ex. 8 |
|---|---|---|---|---|---|---|---|---|---|---|
| Monomer composition (X) | Curable monomer (A) | Bis-GMA | 50 | 50 | 50 | 50 | 50 | | | |
| | | D2.6E | 40 | 40 | 40 | 40 | 40 | 80 | 80 | 80 |
| | | UDMA | | | | | | | | |
| | | DD | 10 | 10 | 10 | 10 | 10 | | | |
| | | 3G | | | | | | 20 | 20 | 20 |
| | | POBMA | | | | | | | | |
| | Polymerization initiator (C) | CQ | 0.4 | 0.4 | 0.4 | 0.4 | 0.4 | 0.2 | 0.2 | 0.2 |
| | | TPO | 0.1 | 0.1 | 0.1 | 0.1 | 0.1 | 0.25 | 0.25 | 0.25 |
| | Polymerization accelerator (E) | PDE | 0.4 | 0.4 | 0.4 | 0.4 | 0.4 | 0.3 | 0.3 | 0.3 |
| | Additive (F) | TIN | 0.3 | 0.3 | 0.3 | 0.3 | 0.3 | 0.3 | 0.3 | 0.3 |
| | | BHT | 0.05 | 0.05 | 0.05 | 0.05 | 0.05 | 0.05 | 0.05 | 0.05 |
| | Refractive index nP of cured product of (A) + (C) + (E) + (F) mixture | | 1.56 | 1.56 | 1.56 | 1.56 | 1.56 | 1.55 | 1.55 | 1.55 |
| | Colorant (D) | D-1 | 0.04982 | 0.16456 | 0.06895 | 0.09502 | 0.04982 | 0.08 | 0.08 | 0.08 |
| | | D-2 | 0.00268 | 0.00507 | 0.00123 | 0.00230 | 0.00268 | 0.002 | 0.002 | 0.002 |
| | | D-3 | 0.00613 | 0.00578 | 0.00381 | 0.00451 | 0.00613 | 0.012 | 0.012 | 0.012 |
| | | D-4 | 0.00349 | 0.0025 | 0.00450 | 0.00288 | 0.00349 | 0.032 | 0.032 | 0.032 |
| | | Colorant (D) in total | 0.06212 | 0.17791 | 0.07849 | 0.10471 | 0.062122 | 0.126 | 0.126 | 0.126 |
| Composition | Monomer composition (X) | | 23 | 23 | 23 | 37.0 | 23 | 30 | 30 | 30 |
| | Inorganic fine particle (BF-1) | BF-1-1 | 43 | 43 | 43 | 63.0 | | | | |
| | | BF-1-2 | | | | | | | | |
| | | BF-1-3 | | | | | | 66 | | 70 |
| | | BF-1-4 | | | | | | | 66 | |
| | | BF-1-5 | | | | | | | | |
| | Additional inorganic agglomerated particle (BF-2e) | BF-2e-1 | | | | | | | | |
| | Light diffusive inorganic agglomerated particle (BF-2d) | BF-2d-1 | | | | | | 4 | 4 | |
| | Light diffusive organic-inorganic composite filler (BC-d) | BC-d-1 | 34 | 34 | | | 34 | | | |
| | | BC-d-2 | | | | | | | | |
| | Additional organic-inorganic composite filler (BC-e) | BC-e-1 | | | | 34 | | | | |
| | Additional inorganic particle (BF-3) | BF-3-1 | | | | | 43 | | | |
| | | Total | 100 | 100 | 100 | 100 | 100 | 100 | 100 | 100 |
| | Refractive index nFBF-2d of light diffusive inorganic agglomerated particle | | - | - | - | - | - | 1.44 | 1.44 | - |
| | Refractive index nFBC-d of light diffusive organic-inorganic composite filler | | 1.50 | 1.50 | 1.50 | - | 1.50 | - | - | - |
| | |nP-nFBF-2d| or |nP-nFBC-d| | | 0.06 | 0.06 | 0.06 | - | 0.06 | 0.11 | 0.11 | - |

[Table 1] Continued

| | | Components (parts by mass) | Ex. 9 | Ex. 10 | Com. Ex. 1 | Com. Ex. 2 | Com. Ex. 3 | Com. Ex. 4 | Com. Ex. 5 |
|---|---|---|---|---|---|---|---|---|---|
| Monomer composition (X) | Curable monomer (A) | Bis-GMA | | | 50 | 50 | 50 | | |
| | | D2.6E | 35 | 35 | 40 | 40 | 40 | 35 | 35 |
| | | UDMA | 10 | 10 | | | | 10 | 10 |
| | | DD | | | 10 | 10 | 10 | | |
| | | 3G | 25 | 25 | | | | 25 | 25 |
| | | POBMA | 30 | 30 | | | | 30 | 30 |
| | Polymerization initiator (C) | CQ | 0.5 | 0.5 | 0.4 | 0.4 | 0.4 | 0.5 | 0.5 |
| | | TPO | 0.4 | 0.4 | 0.1 | 0.1 | 0.1 | 0.4 | 0.4 |
| | Polymerization accelerator (E) | PDE | 0.5 | 0.5 | 0.4 | 0.4 | 0.4 | 0.5 | 0.5 |
| | Additive (F) | TIN | 0.5 | 0.5 | 0.3 | 0.3 | 0.3 | 0.5 | 0.5 |
| | | BHT | 0.05 | 0.05 | 0.05 | 0.05 | 0.05 | 0.05 | 0.05 |
| | Refractive index $nP$ of cured product of (A) + (C) + (E) + (F) mixture | | 1.55 | 1.55 | 1.56 | 1.56 | 1.56 | 1.55 | 1.55 |
| | Colorant (D) | D-1 | 0.082 | 0.086 | 0.15231 | 0.05163 | 0.00036 | - | - |
| | | D-2 | - | 0.0012 | 0.00195 | 0.01676 | 0.01434 | - | - |
| | | D-3 | 0.0032 | 0.0035 | 0.01152 | 0.02448 | 0.097 | - | - |
| | | D-4 | 0.0024 | 0.0031 | 0.00435 | 0.00412 | 0.00792 | - | - |
| | | Colorant (D) in total | 0.0876 | 0.0938 | 0.17013 | 0.09699 | 0.11962 | 0 | 0 |
| Composition | Monomer composition (X) | | 22.5 | 28 | 23 | 23 | 23 | 22.5 | 22.5 |
| | Inorganic fine particle (BF-1) | BF-1-1 | | | 43 | 43 | 43 | | |
| | | BF-1-2 | 20 | 17 | | | | 20 | 20 |
| | | BF-1-3 | | | | | | | |
| | | BF-1-4 | | | | | | | |
| | | BF-1-5 | 5 | | | | | 5 | 5 |
| | Additional inorganic agglomerated particle (BF-2e) | BF-2e-1 | | 50 | | | | | |
| | Light diffusive inorganic agglomerated particle (BF-2d) | BF-2d-1 | | 5 | | | | | |
| | Light diffusive organic-inorganic composite filler (BC-d) | BC-d-1 | | | 34 | 34 | 34 | | |
| | | BC-d-2 | 4 | | | | | | 4 |
| | Additional organic-inorganic composite filler (BC-e) | BC-e-1 | 48.5 | | | | | 52.5 | 48.5 |
| | Additional inorganic particle (BF-3) | BF-3-1 | | | | | | | |
| | | Total | 100 | 100 | 100 | 100 | 100 | 100 | 100 |
| | Refractive index $nF_{BF-2d}$ of light diffusive inorganic agglomerated particle | | - | 1.44 | - | - | - | - | - |
| | Refractive index $nF_{BC-d}$ of light diffusive organic-inorganic composite filler | | 1.49 | - | 1.50 | 1.50 | 1.50 | - | 1.49 |
| | $|nP-nF_{BF-2d}|$ or $|nP-nF_{BC-d}|$ | | 0.06 | 0.11 | 0.06 | 0.06 | 0.06 | - | 0.06 |

[Table 2]

| | | | Ex. 1 | Ex. 2 | Ex. 3 | Ex. 4 | Ex. 5 | Ex. 6 | Ex. 7 | Ex. 8 | Ex. 9 | Ex. 10 | Com. Ex. 1 | Com. Ex. 2 | Com. Ex. 3 | Com. Ex. 4 | Com. Ex. 5 |
|---|---|---|---|---|---|---|---|---|---|---|---|---|---|---|---|---|---|
| Properties | Spectral reflectance ratio [%] | $R_{650/600}$ | 99.9 | 98.8 | 100.3 | 99.6 | 100.1 | 98.9 | 99.0 | 99.1 | 100.1 | 99.7 | 99.1 | 101.1 | 101.8 | 104.0 | 102.5 |
| | | $R_{700/600}$ | 99.8 | 97.9 | 101.5 | 99.5 | 100.6 | 98.5 | 98.6 | 98.3 | 100.7 | 99.1 | 98.0 | 102.3 | 104.2 | 106.8 | 104.2 |
| | | $R_{750/600}$ | 99.7 | 97.1 | 102.8 | 99.8 | 101.2 | 97.8 | 98.0 | 97.3 | 101.3 | 98.5 | 96.8 | 103.5 | 106.1 | 108.1 | 106.3 |
| | Contrast ratio | | 0.48 | 0.54 | 0.48 | 0.46 | 0.49 | 0.53 | 0.53 | 0.47 | 0.45 | 0.41 | 0.56 | 0.51 | 0.48 | 0.23 | 0.34 |
| | Light diffusivity | LD | 0.94 | 0.94 | 0 | 0 | 0.94 | 0.0019 | 0.0019 | 0 | 0.23 | 0.055 | 0.94 | 0.94 | 0.94 | 0 | 0.23 |
| | Lightness | L*/w | 73.54 | 75.68 | 73.84 | 74.61 | 73.57 | 73.45 | 73.96 | 71.2 | 75.22 | 74.21 | 76.79 | 68.6 | 65.89 | 84.2 | 79.4 |
| | Chromaticity | a*/w | -0.44 | -2.5 | -0.21 | -0.27 | -0.5 | 0.01 | 0.03 | 0.13 | -1.67 | -0.2 | -1.19 | -0.75 | 2.31 | -3.7 | -2.5 |
| | | b*/w | 12.82 | 10.51 | 13.01 | 12.81 | 12.67 | 13.59 | 13.71 | 12.51 | 11.06 | 13.55 | 13.44 | 15.9 | 28.91 | 8.4 | 11.1 |
| | Ease of handling of composition | | Good | Good | Good | Moderate | Good | Good | Good | Moderate | Good | Good | Good | Good | Good | Good | Good |
| | Class I color compatibility | ΔE* (A1) | 1.7 | 2.6 | 2.7 | 4.3 | 3.6 | 4.2 | 4.3 | 4.5 | 1.4 | 2.1 | 0.9 | 2.1 | 6.6 | 10.8 | 8.1 |
| | | ΔE* (A3) | 1.9 | 2.1 | 4.3 | 1.7 | 5.5 | 3.4 | 3.1 | 3.8 | 3.6 | 4.1 | 4.8 | 5.1 | 3.7 | 8.2 | 7.5 |
| | | ΔE* (A4) | 2.4 | 5.9 | 5.3 | 4.1 | 5.9 | 2.5 | 2.2 | 3.0 | 4.9 | 3.6 | 7.3 | 7.2 | 2.0 | 7.1 | 5.1 |
| | Class IV color compatibility | ΔE* (A1) | 1.7 | 1.7 | 6.7 | 6.6 | 3.5 | 3.0 | 3.3 | 4.1 | 1.3 | 1.5 | 4.0 | 5.3 | 9.1 | 8.9 | 8.2 |
| | | ΔE* (A3) | 4.6 | 6.8 | 5.2 | 6.1 | 6.6 | 4.5 | 4.1 | 5.6 | 2.2 | 3.7 | 4.1 | 3.5 | 4.5 | 9.3 | 7.2 |
| | | ΔE* (A4) | 5.6 | 7.2 | 9.3 | 5.9 | 7.3 | 5.5 | 5.7 | 6.5 | 2.6 | 5.1 | 8.5 | 9.3 | 2.1 | 9.1 | 6.8 |
| | Ease of polishing [%] | | 33 | 33 | 31 | 35 | 12 | 56 | 57 | 60 | 61 | 44 | 33 | 34 | 33 | 67 | 61 |
| | Gloss retention [%] | | 54 | 53 | 57 | 66 | 45 | 70 | 76 | 73 | 74 | 82 | 54 | 54 | 54 | 75 | 74 |

As shown in Table 2, the dental curable compositions of the present invention, despite being single dental curable compositions, show good color compatibility with natural teeth over a wide range of shades in cavity floors of Class I cavities. It can also be seen that, among these dental curable compositions of the present invention, the dental curable composition of, for example, Example 1 shows high color compatibility also in Class IV cavities because of its light diffusion properties, and, by containing the specific filler (B), has good gloss retention. The color compatibility was inferior in Comparative Examples 1 to 5, in which the spectral reflectance ratios were outside the preferred ranges.

INDUSTRIAL APPLICABILITY

A dental curable composition of the present invention, despite being merely a single composition, shows good color compatibility with natural teeth over a wide range of shades, and has good gloss retention. This makes a dental curable composition of the present invention suitable for use in applications such as dental composite resins.

**Claims**

1. A dental curable composition comprising a polymerizable monomer (A), a filler (B), a polymerization initiator (C), and a colorant (D), wherein the ratios $R_{650/600}$, $R_{700/600}$, and $R_{750/600}$ of spectral reflectances at 650 nm, 700 nm, and 750 nm wavelengths to a spectral reflectance at 600 nm wavelength all fall within a range of 97% to 103% when measured for a 1.0 mm-thick cured product of the dental curable composition against a white background with a spectral colorimeter.

2. The dental curable composition according to claim 1, wherein a 1.0 mm-thick cured product of the dental curable composition satisfies a contrast ratio of 0.35 to 0.65 as defined by the following formula (1),

$$\text{Contrast ratio} = Y_b/Y_w \qquad (1),$$

where $Y_b$ represents a Y value of XYZ color system measured against a black background, and $Y_w$ represents a Y value of XYZ color system measured against a white background.

3. The dental curable composition according to claim 1 or 2, wherein a 0.25 mm-thick cured product of the dental curable composition satisfies a light diffusivity LD of 0.0001 to 0.99 as defined by the following formula (2),

$$LD = (I_5/\cos 5°)/I_0 \qquad (2),$$

where I represents a luminous intensity of light transmitted through a cured product, and $I_0$ and $I_5$ represent luminous intensities of transmitted light at 0- and 5-degree angles, respectively, with respect to a direction perpendicular to a sample plate (a direction of incident light).

4. The dental curable composition according to any one of claims 1 to 3, wherein a 1.0 mm-thick cured product of the dental curable composition has a chromaticity index a*/w of -3.0 to 2.0 as measured in L*a*b*color system with a standard whiteboard placed behind the cured product.

5. The dental curable composition according to any one of claims 1 to 4, wherein the filler (B) comprises an inorganic fine particle (BF-1) having an average particle diameter of 0.05 to 1 $\mu$m.

6. The dental curable composition according to any one of claims 1 to 5, wherein the filler (B) comprises an inorganic agglomerated particle (BF-2) formed by agglomeration of an inorganic primary particle (x), and the inorganic primary particle (x) has an average particle diameter of 0.001 to 1 $\mu$m.

7. The dental curable composition according to claim 6, wherein the inorganic agglomerated particle (BF-2) comprises a light diffusive inorganic agglomerated particle (BF-2d), and the light diffusive inorganic agglomerated particle (BF-2d) has a refractive index that satisfies the following formula (3),

$$0.03 < |nP - nF_{BF-2d}| < 1.0 \qquad (3),$$

where nP represents a refractive index of a polymer obtained by polymerization of the polymerizable monomer (A), and $nF_{BF-2d}$ represents a refractive index of the light diffusive inorganic agglomerated particle (BF-2d).

8. The dental curable composition according to any one of claims 1 to 7, wherein the filler (B) comprises an organic-inorganic composite filler (BC) containing an inorganic primary particle (x), and the inorganic primary particle (x) has an average particle diameter of 0.001 to 1 $\mu$m.

9. The dental curable composition according to claim 8, wherein the organic-inorganic composite filler (BC) comprises a light diffusive organic-inorganic composite filler (BC-d), and the light diffusive organic-inorganic composite filler (BC-d) has a refractive index that satisfies the following formula (7),

$$0.03 < |nP - nF_{BC-d}| < 1.0 \qquad\qquad (7),$$

where nP represents a refractive index of a polymer obtained by polymerization of the polymerizable monomer (A), and $nF_{BC-d}$ represents a refractive index of the light diffusive organic-inorganic composite filler (BC-d).

## INTERNATIONAL SEARCH REPORT

| | International application No. |
|---|---|
| | **PCT/JP2021/039803** |

**A. CLASSIFICATION OF SUBJECT MATTER**

*A61K 6/887*(2020.01)i; *A61K 6/15*(2020.01)i; *A61K 6/16*(2020.01)i; *A61K 6/17*(2020.01)i; *A61K 6/30*(2020.01)i; *A61K 6/60*(2020.01)i; *A61K 6/65*(2020.01)i; *A61K 6/76*(2020.01)i; *A61K 6/84*(2020.01)i
FI: A61K6/887; A61K6/15; A61K6/17; A61K6/16; A61K6/30; A61K6/60; A61K6/65; A61K6/76; A61K6/84

According to International Patent Classification (IPC) or to both national classification and IPC

**B. FIELDS SEARCHED**

Minimum documentation searched (classification system followed by classification symbols)

A61K6/887; A61K6/15; A61K6/16; A61K6/17; A61K6/30; A61K6/60; A61K6/65; A61K6/76; A61K6/84

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2020/138472 A1 (KURARAY NORITAKE DENTAL INC) 02 July 2020 (2020-07-02) examples 1-7, comparative examples 1-4 | 1-9 |
| A | JP 2020-011917 A (TOKUYAMA DENTAL CORP) 23 January 2020 (2020-01-23) comparative example 10 | 1-9 |
| A | WO 2015/064090 A1 (KURARAY NORITAKE DENTAL INC) 07 May 2015 (2015-05-07) examples 1-7, 18-35, comparative examples 1-3, 7-18 | 1-9 |
| A | WO 2019/062144 A1 (LIAONING UPCERA CO., LTD.) 04 April 2019 (2019-04-04) table 1, examples 1-6, comparative examples 7-12 | 1-9 |
| A | WO 2019/189698 A1 (TOKUYAMA DENTAL CORP) 03 October 2019 (2019-10-03) comparative example 8 | 1-9 |
| A | WO 2018/043595 A1 (TOKUYAMA DENTAL CORP) 08 March 2018 (2018-03-08) tables 4-11 | 1-9 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **15 December 2021** | **28 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 238 546 A1

## INTERNATIONAL SEARCH REPORT

International application No.

**PCT/JP2021/039803**

### C. DOCUMENTS CONSIDERED TO BE RELEVANT

| Category* | Citation of document, with indication, where appropriate, of the relevant passages | Relevant to claim No. |
|---|---|---|
| A | JP 2015-067594 A (SHOFU INC) 13 April 2015 (2015-04-13)<br>tables 1-5 | 1-9 |

Form PCT/ISA/210 (second sheet) (January 2015)

35

<table>
<tr><td colspan="2" align="center"><b>INTERNATIONAL SEARCH REPORT</b><br>Information on patent family members</td><td colspan="2">International application No.<br><b>PCT/JP2021/039803</b></td></tr>
</table>

| Patent document cited in search report | Publication date (day/month/year) | Patent family member(s) | Publication date (day/month/year) |
|---|---|---|---|
| WO 2020/138472 A1 | 02 July 2020 | (Family: none) | |
| JP 2020-011917 A | 23 January 2020 | (Family: none) | |
| WO 2015/064090 A1 | 07 May 2015 | US 2016/0250107 A1 examples 1-7, 18-35, comparative examples 1-3, 7-18<br>EP 3064194 A1 | |
| WO 2019/062144 A1 | 04 April 2019 | US 2020/0268616 A1 table 1, examples 1-6, comparative examples 7-12<br>EP 3689322 A1<br>CN 109589270 A<br>KR 10-2020-0060756 A<br>AU 2018340731 A1<br>JP 2020-536061 A | |
| WO 2019/189698 A1 | 03 October 2019 | US 2021/0095113 A1 comparative example 8<br>EP 3777815 A1<br>CN 111902119 A | |
| WO 2018/043595 A1 | 08 March 2018 | US 2019/0192386 A1 tables 4-11<br>EP 3508190 A1<br>CA 3035501 A1<br>AU 2017321653 A1<br>CN 109640924 A<br>KR 10-2019-0046824 A<br>BR 112019003568 A2 | |
| JP 2015-067594 A | 13 April 2015 | US 2015/0094396 A1 tables 1-5<br>EP 2853253 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H09255516 A
- JP S5042696 A
- JP 2011144121 A
- JP 2006510583 T
- WO 2020122192 A

- JP H101473 A
- JP H1192461 A
- JP H093109 A
- JP H10245525 A
- JP 2003096122 A